(19) Europäisches Patentamt
European Patent Office
Office européen des brevets

(11) Numéro de publication : **0 027 089**
**B1**

(12) # FASCICULE DE BREVET EUROPÉEN

(45) Date de publication du fascicule du brevet : **22.01.86**

(51) Int. Cl.[4] : **C 08 B 37/10**, A 61 K 31/725

(21) Numéro de dépôt : **80401425.6**

(22) Date de dépôt : **06.10.80**

(54) **Fractions oligosaccharidiques et oligosaccharides possédant des propriétés biologiques, leurs procédés de préparation et leurs applications en tant que médicaments.**

(30) Priorité : **05.10.79 GB 7934673**
**07.01.80 GB 8000443**
**02.07.80 GB 8021749**
**02.07.80 GB 8021750**
**15.09.80 GB 8029697**

(43) Date de publication de la demande :
**15.04.81 Bulletin 81/15**

(45) Mention de la délivrance du brevet :
**22.01.86 Bulletin 86/04**

(84) Etats contractants désignés :
**AT BE CH DE FR GB IT LI LU NL SE**

(56) Documents cités :
**FR-A- 2 440 376**
**FR-A- 2 461 719**
**GB-A- 2 001 528**
**GB-A- 2 002 406**
**GB-A- 2 035 349**
**US-A- 3 766 167**
**US-A- 4 119 774**
**CHEMICAL ABSTRACTS, vol. 85, no. 13, 27-09-1976, abrégé 89445z, page 239, Columbus, Ohio, US, J.A. CIFONELLI: "Nitrous acid depolymerization of glycosaminoglycans"**
**CHEMICAL ABSTRACTS, vol. 85, no. 17, 25-10-1976, abrégé 118382j, page 190, Columbus, Ohio, US, J.E. SHIVELY et al.: "Formation of anhydrosugars in the chemical depolymerization of heparin"**
**Le dossier contient des informations techniques présentées postérieurement au dépôt de la demande et ne figurant pas dans le présent fascicule.**

(73) Titulaire : **CHOAY S.A.**
**48, Avenue Théophile-Gautier**
**F-75782 Paris Cédex 16 (FR)**

(72) Inventeur : **Lormeau, Jean-Claude**
**23-27 Plein Sud**
**F-76150 Maromme.La Maine (FR)**
Inventeur : **Choay, Jean**
**130, Faubourg Saint-Honoré**
**F-75008 Paris (FR)**
Inventeur : **Petitou, Maurice**
**Appt. 201 27, rue du Javelot**
**F-75645 Paris Cedex 13 (FR)**

(74) Mandataire : **Peaucelle, Chantal et al**
**S.C. Ernest Gutmann - Yves Plasseraud 67, boulevard Haussmann**
**F-75008 Paris (FR)**

**EP 0 027 089 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention est relative à des fractions oligosaccharidiques et à des oligosaccharides possédant des propriétés biologiques leur permettant notamment de contrôler, de manière spécifique, certaines étapes de la coagulation sanguine.

Elle concerne également leurs procédés d'obtention et leurs applications en tant que principes actifs de médicaments.

L'invention est plus particulièrement relative à des oligosaccharides (et aux fractions oligosaccharidiques qui les contiennent) possédant une activité hautement sélective vis-à-vis du facteur X activé ou facteur Xa du sang, à savoir une activité antithrombotique puissante, sans entraîner de risques hémorragiques pour le patient. Le terme « fraction oligosaccharidique » est utilisé dans la description et les revendications pour désigner un mélange relativement homogène de fragments ou chaînes d'oligosaccharides constitués par un nombre variable de motifs saccharidiques.

Les travaux effectués par les inventeurs dans ce domaine les ont amenés à considérer les fractions oligosaccharidiques biologiquement actives, et les oligosaccharides eux-mêmes, tels qu'obtenus à partir de l'héparine.

On notera que le terme héparine est utilisé dans la description et les revendications dans son sens le plus large, pour désigner indifféremment une préparation d'héparine commerciale de qualité pharmaceutique ou une héparine brute telle qu'obtenue par extraction à partir de matériaux biologiques, en particulier de tissus de mammifères.

Il est admis que l'héparine est un polysaccharide hétérogène aussi bien au regard de la composition des chaînes oligosaccharidiques qu'elle comporte que du poids moléculaire de ces chaînes.

Il est également considéré qu'elle renferme principalement des motifs d'acide 2-O sulfate -L-iduronique et de N-sulfate-D-glucosamine (6-O-sulfatée ou non) et, dans une moindre mesure, des motifs d'acide D-glucuronique, d'acide L-iduronique et de N-acétyl-D-glucosamine (6-O-sulfatée ou non).

On sait, en outre que l'héparine exerce son activité anticoagulante en potentialisant l'effet inhibiteur de l'antithrombine III (AT III), qui est une protéine du plasma, vis-à-vis des cascades de réactions enzymatiques mis en jeu au cours de la coagulation.

Etant donné que l'héparine est capable de déprimer simultanément un grand nombre de facteurs de la coagulation intervenant dans la création et le maintien des différentes formes d'hypercoagulabilité, son activité n'apparaît pas spécifique mais globale.

Si cette activité anticoagulante s'avère précieuse, elle rend toutefois délicat le rééquilibrage du système coagulation-fibrinolyse chez les patients en traitement et ce, en raison du caractère global de son action. Il s'ensuit que l'administration (dans le but de prévenir les risques d'hypercoagulation, par exemple l'apparition de thromboses postchirurgicales), de doses trop élevées de médicament anticoagulant, ou l'insuffisante sélectivité de ce médicament, peut finalement être à l'origine d'hémorragies graves.

L'étude approfondie de conditions variées de dépolymérisation de l'héparine et des mélanges de dépolymérisation résultants a amené les inventeurs à constater qu'il est possible, en opérant dans certaines conditions, d'obtenir des mélanges renfermant des oligosaccharides possédant de précieuses propriétés antithrombotiques. Ces oligosaccharides sont plus satisfaisants que l'héparine en ce qui concerne la spécificité de leur activité. Ils sont plus particulièrement capables d'augmenter l'activité spécifique de l'AT III vis-à-vis d'un nombre plus réduit de facteurs de la coagulation, plus spécialement vis-à-vis du facteur Xa du sang. Plus spécialement, les inventeurs ont constaté que de telles fractions et oligosaccharides possèdent une faible activité anticoagulante globale telle que mesurée selon la méthode USP. En conséquence, il apparaît que le rapport de leur activité anti-Xa, exprimée en unités Yin-Wessler et de leur titre USP est élevé ; il est en effet au moins égal à 30.

On rappelle que l'activité Yin-Wessler est plus spécialement représentative de l'aptitude des fractions actives à potentialiser l'inhibition du facteur Xa du sang par l'AT III dans le test correspondant et le titre USP du pouvoir des fractions actives à inhiber la coagulation totale du sang ou du plasma.

Le titre Yin-Wessler est mesuré selon la méthode décrite par ces auteurs dans J. Lab. Clin. Med. 1976, 81, 298 à 300 et le titre U.S.P. selon la méthode décrite dans « Pharmacopea of the United States of America », pages 229 et 230 (voir également le deuxième supplément U.S.P.-NF, page 62 et le quatrième supplément U.S.P., page 90, intitulé respectivement « Drug substances » et « Dosage Forms »).

D'une manière inattendue, il est ainsi apparu que cette activité spécifique anti-Xa souhaitée se retrouvait dans les chaînes oligosaccharidiques courtes, à savoir celles ne renfermant pas plus de 8 motifs, pouvant être isolées des mélanges de dépolymérisation en ayant recours à certaines opérations de purification réalisées dans des conditions déterminées.

On notera que le terme « motif saccharidique » tel qu'utilisé dans la description et les revendications désigne les monosaccharides contenus dans les chaînes de l'héparine.

En outre, selon un aspect de grand intérêt, l'étude de ces fractions et oligosaccharides par les inventeurs a montré que cette activité anti-Xa, telle que mesurée par le test Yin-Wessler, était significative d'une activité antithrombotique *in vivo.*

L'invention vise donc à fournir de nouveaux oligosaccharides et les fractions qui les renferment, possédant une activité anti-Xa élevée et présentant à l'égard du facteur Xa une sélectivité remarquable dans le cadre des réactions enzymatiques successives qui caractérisent le processus de coagulation.

Elle vise également à fournir des caractéristiques de structure de ces oligosaccharides.

Elle a également pour but de fournir un procédé d'obtention de ces fractions, de mise en œuvre aisée.

L'invention a, en outre, pour but, de fournir des principes actifs de médicaments, et les médicaments eux-mêmes, capables notamment d'inhiber le facteur Xa selon un degré de sélectivité élevé alors que leur activité sur la coagulation globale peut être maintenue à un niveau très faible. Ces médicaments sont avantageusement utilisables pour des traitements antithrombotiques sans entraîner de risques hémorragiques.

Les fractions évoquées ci-dessus sont du type de celles pouvant être obtenues par un procédé qui comprend les étapes de :

— mise en contact de l'héparine (ou de fractions hépariniques) possédant une activité anticoagulante et ayant des chaînes de poids moléculaire d'environ 2 000 à environ 50 000, avec un agent capable de dépolymériser ou de fragmenter les chaînes hépariniques, les conditions utilisées pour réaliser cette étape étant ajustées de manière à obtenir un mélange de dépolymérisation contenant des fragments (ou chaînes oligosaccharidiques courtes) constitués par 8 motifs au plus, mais possédant cependant une activité anti-Xa (Yin-Wessler) d'au moins 100 u/mg alors que leur activité anticoagulante globale (titre USP) est faible par rapport à celle de l'héparine, voire pratiquement nulle et possédant une activité élevée pour l'AT III ;

— le contact du mélange de dépolymérisation avec de l'AT III pour absorber ou retenir sélectivement au moins la majeure partie, avantageusement pratiquement la totalité des oligosaccharides possédant une séquence ayant une affinité spécifique pour l'AT III ;

— la récupération des produits fixés sur l'AT III, et l'isolement des fractions recherchées renfermant lesdites chaînes oligosaccharidiques constituées par huit motifs au plus, et les sels pharmaceutiquement acceptables de ces fractions.

Lesdits oligosaccharides peuvent être caractérisés par le fait qu'ils possèdent une structure spécifique capable de se lier à l'AT III. Ils possèdent une activité anti-Xa supérieure à celle de l'héparine et une activité anticoagulante globale très faible.

Les produits possédant une affinité pour l'AT III, tels que récupérés à partir du procédé défini ci-dessus, sont soumis à une ou plusieurs étapes de séparation sélective les oligosaccharides à chaînes courtes définis ci-dessus.

Cette séparation est avantageusement obtenue par fractionnement du mélange des produits élués possédant une affinité pour l'AT III, selon leur poids moléculaire et/ou leur densité ionique, puis par récupération des fractions désirées.

En variante, on peut réaliser le fractionnement directement sur le mélange de dépolymérisation. A ce stade, les fractions comprenant des chaînes oligosaccharidiques courtes, avantageusement les oligosaccharides *per se,* peuvent être isolés. Les fractions, le cas échéant les oligosaccharides, possédant une affinité élevée pour l'AT III, sont ensuite séparées en ayant recours à une étape comprenant la mise en contact de ces fractions avec de l'AT III comme indiqué ci-dessus.

Les fractions oligosaccharidiques de l'invention sont du type de celles obtenues en ayant recours aux différentes étapes définies ci-dessus. Elles sont caractérisées par le fait qu'elles sont dépourvues d'oligosaccharides comprenant plus de 8 motifs saccharidiques et qu'elles sont constituées par des oligosaccharides ne renfermant pas plus de 8 motifs saccharidiques contenant une séquence comportant moins de 8 motifs saccharidiques, responsable, au moins, dans une large mesure, de leur activité anti-Xa.

Selon un mode de réalisation, les fractions de l'invention sont du type de celles pouvant être obtenues par un procédé dans lequel le matériau héparinique est dépolymérisé par un procédé chimique, en particulier de l'acide nitreux $HNO_2$ en milieu aqueux.

Les chaînes hépariniques sont alors coupées entre un motif N-sulfate glucosamine et le motif acide uronique suivant, ce qui entraîne la formation de chaînes contenant un groupe 2,5-anhydromannose à leur extrémité réductrice.

Dans un autre mode de réalisation, la dépolymérisation est réalisée par voie enzymatique, avantageusement avec une héparinase hautement purifiée, plus particulièrement une héparinase d'origine bactérienne.

Cette enzyme provoque la coupure des chaînes hépariniques entre le carbone anomère d'un résidu N-sulfate-glucosamine et le motif acide uronique suivant.

Son action produit un mélange d'oligosaccharides (di-, tetra-, hexa-, octasaccharides) ayant essentiellement un nombre pair de motifs saccharidiques et terminés à leur extrémité non réductrice par un acide uronique $\alpha,\beta$-insaturé.

On réalise avantageusement la dépolymérisation avec l'héparine purifiée dans des conditions permettant d'obtenir lesdits oligosaccharides biologiquement actifs, ne renfermant pas plus de 8 motifs saccharidiques et, pour certains d'entre eux, pas plus de 6 ou même moins.

Ces conditions sont telles que l'on atteigne la limite de la réaction enzymatique. En d'autres termes, les oligosaccharides résultants ne sont plus sensibles à l'action de l'héparinase hautement purifiée.

Les oligosaccharides biologiquement actifs correspondent alors à ceux qui peuvent être séparés desdits mélanges de dépolymérisation (obtenus par voie chimique ou enzymatique), par adsorption sur

de l'AT III fixée sur un support par exemple de l'AT III fixée sur de l'agarose, dans des conditions permettant la fixation des produits ayant une affinité pour l'AT III, tandis que ceux qui sont dépourvus d'une telle affinité sont éliminés, par exemple par lavage.

A la suite de cette opération, on procède à l'élution des produits retenus ou adsorbés afin de les récupérer et à leur fractionnement pour isoler les chaînes courtes ayant une activité anti-Xa.

Le cas échéant, avant cette séparation basée sur l'affinité des fractions pour l'AT III, on soumet le mélange de dépolymérisation à un fractionnement comme déjà mentionné, afin de séparer les chaînes désirées. On effectue avantageusement une telle séparation par gel perméation (gel filtration).

La plupart des chaînes oligosaccharidiques telles que celles isolées ci-dessus, outre leur affinité élevée pour l'AT III, et par suite leur activité anti-Xa, sont caractérisées par des spectres RMN comprenant, entre autres, un signal caractéristique dans la région du carbone anomère en position 2 des résidus N-sulfate-glucosamine (ce signal étant repéré par un astérisque sur les fig. 5 et 6). Ce signal n'apparaît pas avec l'héparine. Il peut être vraisemblablement attribué à la présence d'un substituant sur l'atome d'oxygène en position 3, et plus particulièrement à un groupe 3-O-sulfate, sur le résidu N-sulfate-D-glucosamine.

Ces oligosaccharides sont, en outre, caractérisés par un titre Yin-Wessler et un titre USP qui sont dans un rapport respectivement d'au moins 30, de préférence d'au moins 100.

Des fractions oligosaccharidiques préférées et des oligosaccharides comprennent ceux ayant une affinité pour l'AT III et une activité anti-Xa (Yin-Wessler) supérieure à celle de l'héparine. Une telle activité peut être 10 fois supérieure à celle de l'héparine.

Des activités supérieures à 100 u/mg peuvent être observées pour certains oligosaccharides. Des valeurs supérieures à 700 u/mg, ou encore d'au moins 1 000 u/mg, même 1 200, pouvant atteindre 2 000 u/mg ou plus ont été observées pour d'autres oligosaccharides.

Des oligosaccharides actifs de l'invention possèdent un résidu N-sulfate-D-glucosamine avantageusement 3-O-sulfaté (désigné par F dans la formule donnée ci-après).

D'autres oligosaccharides comprennent, en outre, un résidu N-acétyl-D-glucosamine (D) et/ou un résidu d'acide D-glucuronique (E) et/ou un résidu d'acide 2-O-sulfate-L-iduronique (G) et/ou un résidu de N-sulfate-D-glucosamine (H).

Chez d'autres oligosaccharides, on observe la présence des motifs saccharidiques suivants, à savoir d'acide 2-O-sulfate, 4,5-insaturé uronique (A) et/ou de N-sulfate-D-glucosamine (B) et/ou d'acide L-iduronique (C).

Des oligosaccharides préférés de l'invention possèdent un résidu N-sulfate-D-glucosamine à leur extrémité réductrice. Ce résidu est sulfaté ou non en position 3 et/ou 6.

Certains oligosaccharides contiennent tous les résidus indiqués ci-dessus.

Le dosage par colorimétrie des motifs N-acétyl-glucosamine, selon la méthode de Smith et Gilkerson dans Annal. Biochem. 1979, 98, p. 478-480, montre alors la présence d'environ une molécule de N-acétyl glucosamine par chaîne oligosaccharidique.

Le dosage de glucosamine avant et après hydrolyse acide permet la détermination des quantités respectives des motifs N-sulfate-glucosamine et N-acétyl-glucosamine.

Certains oligosaccharides de l'invention apparaissent ainsi caractérisés par la présence d'un motif N-acétyl-D-glucosamine pour deux motifs N-sulfate-glucosamine.

D'autres oligosaccharides sont caractérisés par la présence d'un motif N-acétyl-D-glucosamine pour trois motifs N-sulfate-D-glucosamine.

Un oligosaccharide de ce type est constitué par un octasaccharide correspondant à celui présentant la séquence suivante ABCDEFGH, dans laquelle les différents symboles présentent les significations suivantes :

A = acide uronique ou acide uronique insaturé,

B = N-sulfate-D-glucosamine ou N-sulfate 6-O-sulfate D glucosamine,

C = acide L-iduronique, le cas échéant, lorsque ce motif se trouve du côté de l'extrémité non réductrice, un acide iduronique insaturé,

D = N-acétyl D-glucosamine ou N-acétyl 6-O-sulfate D-glucosamine,

E = acide D-glucuronique

F = N-sulfate 3-O-sulfate D-glucosamine ou N-sulfate 3-O-sulfate 6-O-sulfate D-glucosamine,

G = acide 2-O sulfate L-iduronique et

H = N-sulfate D-glucosamine ou N-sulfate 6-O sulfate D-glucosamine.

(Voir dessin p. 5)

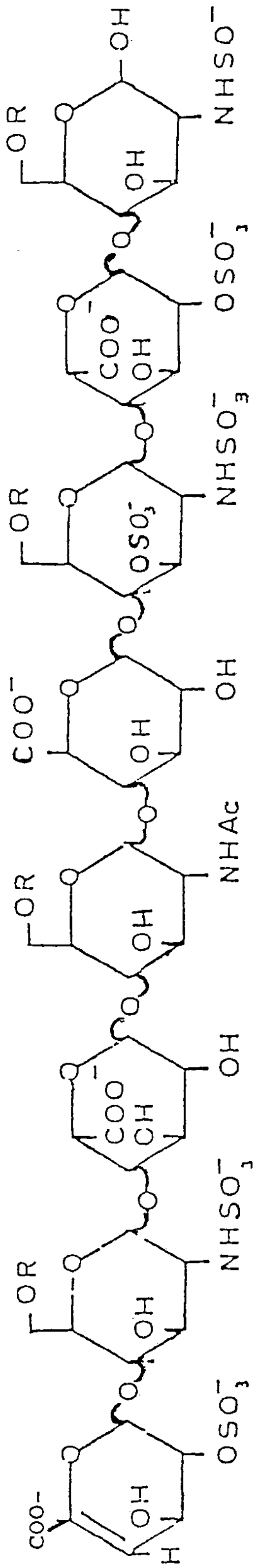

A B C D E F G H

Dans cette formule, R représente un atome d'hydrogène ou un groupe sulfate $SO_3^-$.

Dans un octasaccharide de structure ABDCEFGH, A est un acide glucuronique et le groupe terminal est un groupe 2,5-anhydromanno. A est notamment un motif glucuronique insaturé.

Un autre octasaccharide possède la séquence ABGHCDEF.

Des hexasaccharides actifs de l'invention comprennent également des motifs parmi lesdits motifs A, B, C, E, et H. L'un de ces hexasaccharides possède la séquence ABCDEF.

Un autre hexasaccharide possède la séquence CDEFGH où C est un acide uronique insaturé.

Encore un autre hexasaccharide possède la séquence CDEFGH (où C est un résidu d'acide iduronique).

D'autres oligosaccharides actifs sont des pentasaccharides, en particulier celui possédant la structure DEFGH.

Des chaînes oligosaccharidiques plus courtes, encore biologiquement actives, font également partie de l'invention.

L'invention vise également les sels pharmaceutiquement acceptables de ces oligosaccharides, plus spécialement les sels de sodium, de calcium ou de magnésium acceptables de ces oligosaccharides.

L'invention vise également un procédé d'obtention des fractions et oligosaccharides évoqués ci-dessus, comportant la dépolymérisation de l'héparine et le traitement du mélange de dépolymérisation résultant afin d'en séparer les fractions renfermant des oligosaccharides constitués au plus par huit motifs saccharidiques (avantageusement ces oligosaccharides eux-mêmes) possédant une affinité pour l'AT III, une activité anti-Xa (Yin-Wessler) élevée et un titre USP très faible.

Ce procédé comprend :

a) la mise en contact de l'héparine (ou de fractions hépariniques) possédant une activité anti-coagulante et comportant des chaînes avec des poids moléculaires d'environ 2 000 à environ 50 000, avec un agent capable de dépolymériser ou de fragmenter les chaînes hépariniques dans des conditions ajustées afin d'obtenir un mélange de dépolymérisation qui contient des fragments ou des chaînes oligosaccharidiques constituées par huit motifs au plus, possédant cependant une activité anti-Xa (Yin-Wessler) d'au moins 100 u/mg alors que leur activité anticoagulante globale est faible par rapport à celle de l'héparine, voire nulle, et une affinité spécifique élevée pour l'AT III ;

b) la mise en contact du mélange de dépolymérisation avec de l'AT III pour absorber ou retenir au moins la majeure partie des oligosaccharides possédant une séquence ayant une affinité spécifique vis-à-vis de l'AT III,

c) la désorption des produits retenus sur l'AT III et la récupération des fractions et oligosaccharides recherchés.

L'étape de dépolymérisation est réalisée dans des conditions ménagées afin de ne pas dégrader complètement les chaînes oligosaccharidiques et de conserver les motifs responsables, dans une large mesure, de l'activité anti-Xa (Yin-Wessler).

De préférence, les conditions de dépolymérisation du procédé défini ci-dessus sont ajustées de manière à conserver le pouvoir d'au moins une partie des oligosaccharides résultants d'être retenus spécifiquement sur de l'AT III immobilisée.

Ces conditions sont choisies de manière à conserver une activité anti-Xa (mesurables par le test Yin-Wessler) chez au moins une partie des oligosaccharides résultants, leur activité anti-coagulante selon le test USP étant pratiquement nulle.

Naturellement, les conditions de dépolymérisation données ci-dessus peuvent être modifiées, elles apparaissent toutefois avantageuses lorsqu'on souhaite obtenir des rendements élevés en oligosaccharides possédant une activité anti-Xa (Yin-Wessler).

Lorsqu'on dépolymérise l'héparine avec $HNO_2$, il apparaît avantageux de réaliser la réaction en milieu aqueux, à un pH de l'ordre de 2 à 4, de préférence de 3, et à une température voisine de l'ambiante.

Lorsqu'on dépolymérise avec une héparinase, on met en œuvre de préférence une héparinase hautement purifiée, en particulier, une héparinase d'origine bactérienne, plus spécialement provenant de Flavobacterium heparinum. On contrôle les conditions afin d'obtenir les fragments les plus courts possédant encore une affinité pour l'AT III et une activité anti-Xa (Yin-Wessler).

On opère avantageusement à un pH de l'ordre de 6 à 8, en particulier voisin de la neutralité et à une température voisine de l'ambiante. On ajoute l'héparinase portion par portion dans le mélange réactionnel jusqu'à hydrolyse complète.

La dépolymérisation avec l'héparinase peut être, le cas échéant, effectuée sur les fractions résultant des fractions oligosaccharidiques obtenues après dégradation de l'héparine par $HNO_2$. On peut également la réaliser avec les oligosaccharides de poids moléculaire élevé qui sont retenus, sur l'AT III immobilisée, en même temps que les oligosaccharides actifs comportant moins de 8 motifs, et qui sont élués avec ces oligosaccharides à chaînes courtes.

La séparation et la récupération de fractions contenant les oligosaccharides désirés sont avantageusement réalisées par chromatographie d'affinité dans une colonne contenant de l'AT III immobilisée.

Des résultats satisfaisants sont obtenus en ayant recours à un gel d'agarose commercialisé sous la dénomination Sépharose sur lequel sont immobilisés des motifs d'AT III.

Pour obtenir la séparation désirée de la majeure partie des produits contenus dans le mélange de dépolymérisation et ayant une affinité élevée pour l'AT III, on équilibre avantageusement la colonne avec

un tampon ayant une force ionique d'environ 0,2 M, de préférence, supérieure à 0,1 M, à un pH de 6 à 8, de préférence, voisin ou légèrement supérieur à la neutralité.

Les produits dépourvus d'affinité pour l'AT III ou n'ayant qu'une très faible affinité pour l'AT III sont éliminés par lavage avec un tampon avantageusement du même type que celui utilisé pour l'équilibrage de la colonne.

Un mode de réalisation préféré pour récupérer les produits retenus ou absorbés sur l'AT III, ayant une activité anti-Xa (Yin-Wessler), comprend la désorption et la récupération de tous les oligosaccharides par élution avec un tampon ayant une force ionique suffisante à cet effet.

Le tampon utilisé pour cette élution est alors avantageusement choisi parmi ceux qui n'interfèrent pas avec les étapes de récupération ultérieure (en particulier avec la précipitation alcoolique) des oligosaccharides contenus dans les fractions récupérées.

Un tampon approprié contient un sel de calcium, tel que le chlorure de calcium qui reste soluble en présence d'une concentration en alcool (tout alcool approprié, par exemple l'éthanol) provoquant la précipitation des oligosaccharides. On peut également utiliser un sel de sodium.

Après élution des produits à affinité pour l'AT III, il est avantageux d'effectuer une précipitation alcoolique aux fins de leur récupération, leur séparation étant ensuite effectuée, par exemple, par centrifugation.

Afin d'obtenir des fractions d'oligosaccharides ayant une forte activité anti-Xa (Yin-Wessler) et un caractère homogène satisfaisant, on soumet le mélange de tous les oligosaccharides initialement retenus sur l'AT III à une opération de fractionnement, avantageusement par gel filtration, ou encore par chromatographie d'échange d'ions ou les deux ou toute autre méthode qui conduirait à des résultats similaires.

On conduit avantageusement l'opération de gel filtration de manière à éluer tout d'abord les plus grosses molécules puis à récupérer les plus petites en commençant avec les fractions qui ont un titre Yin-Wessler et un titre USP dans un rapport d'au moins 30 ou même d'au moins 50, de préférence, de 100 ou supérieur, la récupération s'étendant à toutes les fractions restantes qui possèdent encore une activité anticoagulante au moins dans le test de Yin-Wessler.

Naturellement, selon les quantités de solution soumises à l'opération de gel filtration, on choisira les volumes des fractions successivement éluées afin de retenir celles des fractions les plus appropriées pour l'application souhaitée.

Dans une variante, avant l'étape de fixation sur l'AT III, on élimine du mélange de dépolymérisation les oligosaccharides comportant plus de 8 motifs saccharidiques et ce, avantageusement par gel filtration ou une technique similaire comme indiqué ci-dessus.

A partir des fractions éluées ci-dessus, on peut séparer des oligosaccharides biologiquement actifs, à savoir des octa-, hepta-, hexa-, penta-, tetra-, et des trisaccharides.

L'étude pharmacologique des fractions et oligosaccharides de l'invention a montré qu'il existe un rapport entre leur activité anti-Xa telle qu'exprimée en unités Yin-Wessler et leur activité antithrombotique.

Ces fractions et oligosaccharides apparaissent capables d'exercer une activité antithrombotique puissante. En raison de leur activité anticoagulante faible et même pratiquement nulle, les risques d'hémorragie sont avantageusement pratiquement éliminés.

Parmi les essais effectués *in vivo*, pour étudier leur activité antithrombotique, le test suivant a été réalisé sur le lapin selon la méthode de Wessler et al. dans J. of appr. Physiol 1959, 14, 943-946.

On a provoqué la formation de caillots dans la veine jugulaire du lapin en injectant un complexe de prothrombine activée.

On a étudié l'effet préventif de l'octosaccharide ABCDEFGH, possédant un titre Yin-Wessler de 2 000 unités par mg, vis-à-vis de la formation de caillots et ce, en injectant l'octasaccharide avant injection de 25 unités/kg de complexe thrombogénique.

Lorsqu'on injecte les oligosaccharides ayant le complexe de prothrombine activée à des doses de 150 à 250 u/mg (Yin-Wessler), on obtient une protection importante vis-à-vis de la formation de caillots.

Cet octasaccharide possède donc une activité antithrombotique puissante. Avantageusement, on ne peut déceler d'activité anticoagulante globale.

Les fractions oligosaccharidiques de l'invention sont dépourvues de toxicité. L'administration de 10 000 u/kg (titre Yin-Wessler) de l'une des fractions de l'invention ne provoque chez le lapin aucune réaction toxique, ni d'effet pyrogénique dans le test de pyrogénicité chez le lapin conforme à la pharmacopée française.

Les compositions selon l'invention sont alors particulièrement appropriées pour le contrôle spécifique de certaines étapes de la coagulation chez l'homme ou l'animal, en particulier, lorsqu'on souhaite effectuer un contrôle sélectif de l'activité du facteur Xa du sang (par exemple chez les patients qui ont subi, ou qui vont subir une opération, dans le cas de maladies athéromateuses, de perturbations de mécanismes de la coagulation par des activateurs bactériens ou enzymatiques etc...).

L'invention est donc relative à des préparations pharmaceutiques qui renferment lesdites fractions oligosaccharidiques ou les oligosaccharides eux-mêmes, à activité anti-Xa élevée.

Elle est plus particulièrement relative à des préparations pharmaceutiques dépourvues de substances pyrogéniques contenant une quantité efficace de principes actifs en association avec des excipients pharmaceutiques.

En particulier, elle concerne les compositions dans lesquelles le véhicule pharmaceutique est approprié pour l'administration par voie orale. Des formes d'administration de l'invention appropriées pour l'administration par voie orale peuvent être avantageusement des gélules gastrorésistantes, des comprimés ou tablettes ou des pilules.

D'autres compositions pharmaceutiques comprennent ces oligosaccharides ou fractions oligosaccharidiques en association avec les excipients appropriés pour l'administration par voie rectale. Des formes d'administration correspondantes sont constituées par des suppositoires.

D'autres formes d'administration de l'invention sont constituées par des aérosols ou des pommades.

L'invention concerne également des compositions pharmaceutiques injectables, stériles ou stérilisables.

Ces solutions renferment avantageusement 1 000 à 100 000 u (Yin-Wessler)/ml d'oligosaccharides ou de fractions oligosaccharidiques, de préférence de 5 000 à 50 000, par exemple de 25 000 u/ml, lorsque ces solutions sont destinées à l'injection par voie sous-cutanée. Elles peuvent contenir, par exemple, de 500 à 10 000, notamment 5 000 u/ml d'oligosaccharides ou de fractions oligosaccharidiques lorsqu'elles sont destinées à l'injection par voie intraveineuse ou par perfusion.

Avantageusement, de telles préparations pharmaceutiques sont présentées sous la forme de seringues non récupérables, prêtes à l'emploi.

L'invention concerne également les compositions pharmaceutiques contenant lesdits oligosaccharides en association avec un autre principe actif, utilisable en particulier pour la prophylaxie et le traitement de thrombose, tel qu'un agent veinotonique comme la dihydroergotamine, un sel d'acide nicotinique ou un agent thrombolytique comme l'urokinase.

Les fractions oligosaccharidiques et oligosaccharides de l'invention sont avantageusement sous la forme de sels d'au moins un métal physiologiquement acceptable tel que le sodium et/ou le calcium et/ou le magnésium.

Les compositions pharmaceutiques de l'invention sont particulièrement adaptées pour le contrôle (préventif ou curatif) de certaines étapes de la coagulation du sang chez l'homme ou l'animal, notamment dans le cas où le patient est soumis à des risques d'hypercoagulabilité résultant notamment de la libération par l'organisme de thromboplastines, par exemple, de thromboplastines tissulaires (opérations chirurgicales, processus athéromateux, développement de tumeurs et troubles de la coagulation par des activateurs bactériens ou enzymatiques, etc...).

Afin d'illustrer l'invention, on indique, ci-après, un exemple de posologie utilisable chez l'homme : cette posologie comprend, par exemple, l'administration au patient de 1 000 à 25 000 u par voie sous-cutanée, deux ou trois fois par jour, selon le niveau des risques d'hypercoagulabilité ou la condition thrombotique du patient, ou de 1 000 à 25 000 u/24 heures par voie intraveineuse, en administrations discontinues à intervalles réguliers, ou continues par perfusion, ou encore de 1 000 à 25 000 u (trois fois par semaine) par voie intramusculaire (ces titres sont exprimés en unités Yin-Wessler). Ces doses peuvent être naturellement ajustées pour chaque patient en fonction des résultats et des analyses de sang effectuées auparavant, la nature des affections dont il souffre et, d'une manière genérale, son état de santé.

L'invention se rapporte également à l'application des oligosaccharides de l'invention et des fractions qui les renferment, à la constitution de réactifs biologiques, utilisables en laboratoire notamment comme éléments de comparaison pour l'étude d'autres substances dont on souhaite tester l'activité anticoagulante, notamment au niveau de l'inhibition du facteur Xa.

Elle vise également l'application des fractions et oligosaccharides en médecine nucléaire, en tant que produits radiopharmaceutiques. Les oligosaccharides et fractions définis ci-dessus sont marqués par des traceurs choisis parmi ceux couramment utilisés dans ce domaine, et notamment à l'aide de technétium 99 m.

A cet effet, on transforme le technétium 99 m obtenu à partir de générateurs du commerce, sous forme de pertechnétate de sodium de valence 7 non réactif, en technétium réduit de valence 4 qui serait la forme la plus réactive du technétium. Cette transformation est effectuée grâce à un système réducteur réalisé à partir de sels d'étain (chlorure stanneux), de sels de fer (sulfate ferreux), de sels de titane (trichlorure de titane) ou d'autres sels.

La plupart du temps, cette simple réduction du technétium suffit, dans des conditions de pH donné, à réaliser la fixation du technétium sur la molécule considérée.

On peut utiliser les produits de l'invention, qui constituent en quelque sorte un support, à des doses de l'ordre de 100 à 200 u Yin-Wessler.

Pour l'élaboration de ces radiopharmaceutiques, on peut opérer conformément à la méthode de P.V KULKARNI et *al.* dans The Journal of Nuclear Medecine 21, No 2, p. 117-121.

Les produits ainsi marqués sont avantageusement utilisés dans des tests in vivo pour la détection et le diagnostic d'extension des thromboses et des états thrombotiques.

D'autres caractéristiques et avantages de l'invention apparaîtront dans la description qui suit des exemples et en se référant aux dessins.

Exemple 1

Procédé d'obtention de fractions oligosaccharidiques comprenant les étapes de :

I. Dépolymérisation d'héparine avec $HNO_2$,

II. Séparation des oligosaccharides biologiquement actifs par chromatographie sur Sépharose-AT III,

III. Gel filtration des fractions éluées et récupération des produits désirés.

I. Dépolymérisation d'héparine en présence d'acide nitreux

On dissout 20 g d'héparine (titre USP : 150 UI/mg, titre YIN-WESSLER : 150 UI/mg) dans 800 ml d'eau à température ambiante.

On ajoute 200 ml d'acide sulfurique 0,5 M puis 13,8 g de nitrite de sodium (moralité finale de l'acide sulfurique : 0,1 M, et du nitrite : 0,2 M).

La réaction s'accompagne d'un dégagement d'azote gazeux. On arrête la réaction au bout de 15 minutes en ajustant le pH à 7-7,2 avec de la soude 5 N.

Les produits de dépolymérisation sont précipités par de l'éthanol (7 volumes), à savoir, dans ce cas, 7 litres.

Le précipité est centrifugé, lavé avec de l'alcool et séché sous vide.

On récupère 24,8 g de produit ayant un titre USP nul et un titre YIN-WESSLER de 7 UI/mg.

L'augmentation aparente de poids du précipité résulte de la précipitation partielle du sulfate de sodium avec les produits de dépolymérisation.

II. Chromatographie sur SEPHAROSE-AT III

Les produits de dépolymérisation obtenus sont soumis à une chromatographie sur gel de SEPHAROSE sur lequel sont retenus des motifs d'AT III.

Avec un tampon constitué par NaCl 0,2 M, tris-HCl 0,05 M, pH 7,2, on équilibre une colonne (de 2,6 cm de diamètre et de 40 cm de hauteur) contenant 200 ml de SEPHAROSE- AT III (approximativement 10 mg d'AT III bovine immobilisée par ml de SEPHAROSE).

On dissout 1,6 g des produits de dépolymérisation ci-dessus dans 16 ml d'un tampon NaCl 0,2 M et on fait passer cette solution à travers une colonne à une vitesse de 50 ml par heure, puis on rince la colonne avec 500 ml d'un tampon NaCl 0,2 M.

Les oligosaccharides retenus sont ensuite élués de la colonne à l'aide d'un tampon capable de désorber tous les oligosaccharides retenus sur l'AT III immobilisée ($CaCl_2$ 0,2 M, tris-HCl « tris » représente la tris-(hydroxyméthyl)-méthylamine 0,05 M, pH 7,2). On récupère la partie de l'effluent qui contient les oligosaccharides, et on précipite ces oligosaccharides à l'aide de 10 volumes d'éthanol.

Après lavage avec de l'éthanol et séchage sous vide, on récupère 5 mg de produit ayant un titre USP de 66 UI/mg et un titre YIN WESSLER de 1 600 UI/mg.

Les produits ainsi obtenus sont constitués par un mélange de mucopolysaccharides de poids moléculaire élevé et d'oligosaccharides de faible poids moléculaire.

III. Gel filtration

On dissout dans 2 ml d'eau distillée 90 mg d'un mélange de mucopolysaccharides et d'oligosaccharides obtenus par un procédé dont les paragraphes précédents sont représentatifs. On dépose cette solution au sommet d'une colonne (hauteur 1 m, diamètre 2,6 cm) de gel filtration formée de particules extrêmement fines commercialisées sous la dénomination SEPHADEX G 50, cette colonne étant équilibrée avec de l'eau distillée. On réalise l'élution avec de l'eau distillée à une vitesse de 26 ml/h. On recueille l'effluent par fractions (6 ml par tube). On évalue la teneur en mucopolysaccharides de chaque tube par absorption UV à 206 nanomètres.

Les contenus des tubes sont réunis pour former les fractions numérotées 1 à 5.

Les mucopolysaccharides contenus dans chacune de ces fractions sont précipités par de l'alcool et séchés.

On indique ci-après les poids et les propriétés anticoagulantes de ces fractions :

| | | |
|---|---|---|
| fraction (1) | Poids | 15 mg |
| | Titre USP | 156 UI/mg |
| | Titre YIN-WESSLER | 190 U/mg |
| fraction (2) | Poids | 16 mg |
| | Titre USP | 139 UI/mg |
| | Titre YIN-WESSLER | 520 U/mg |
| fraction (3) | Poids | 17 mg |
| | Titre USP | 50,3 UI/mg |
| | Titre YIN-WESSLER | 1 390 U/mg |

| | | | |
|---|---|---|---|
| fraction (4) | Poids | 40 | mg |
| | Titre USP | 7 | UI/mg |
| | Titre YIN-WESSLER | 870 | U/mg |
| fraction (5) | Poids | 1 | mg |
| | Titre USP | nul | |
| | Titre YIN-WESSLER | 150 | U/mg |

On étudie les domaines de poids moléculaire des fractions ainsi obtenues, en particulier de la « fraction 4 » et de la « fraction 5 », à la fois par chromatographie sur papier par Chromatographie Liquide sous Haute Pression (HPLC) et on effectue une étude comparative en considérant les résultats obtenus selon les mêmes conditions expérimentales avec un décasaccharide d'héparine et :

— par chromatographie sur papier, avec les produits de référence décrits dans l'article de SILVA et DIETRICH (SILVA M. E. et DIETRICH C. P., J. Biol. chem. Vol. 250 (1975) p. 6841-6846), et

— par HPLC sur gel de silice, avec une série de polystyrène-sodium sulfonates ayant des poids moléculaires connus allant en augmentant et des échantillons d'héparine de poids moléculaires connus.

La « fraction 4 » apparaît constituée par un oligosaccharide possédant moins de 8 motifs saccharidiques et probablement pas plus de 6 motifs saccharidiques, et la « fraction 5 » d'un oligosaccharide possédant moins de 6 motifs saccharidiques, plus vraisemblablement pas plus de 4 motifs saccharidiques.

## Exemple 2

Procédé d'obtention de fractions oligosaccharidiques comprenant les étapes de :

A. Dépolymérisation de l'héparine par digestion avec une héparinase ;

B. Fractionnement du mélange de dépolymérisation par gel filtration sur DEAE Sephadex A 25 ;

C. Isolement des produits biologiquement actifs par chromatographie sur agarose — AT III.

### A. Dépolymérisation de l'héparine par digestion avec une héparinase

1. Préparation de l'héparinase :

Pour la dégradation de l'héparine on utilise des enzymes de Flavobacterium heparinum.

Les enzymes de Flavobacterium heparinum sont cultivés selon la méthode de Payza et Korn, décrite dans J. Biol. Chem. 1956, 223, p. 853-858. Les cellules lyophilisées sont broyées à sec en présence d'alumine puis extraites par un tampon acétate à pH neutre.

Les parties insolubles sont éliminées et la solution obtenue est successivement chromatographiée sur DEAE puis sur deux agaroses tels que ceux commercialisés sous les dénominations ® CM Sepharose CL 6B et ® Ultrogel ACA 54.

On obtient ainsi 20 mg d'héparinase ayant un degré de pureté de 90 % (cette pureté est évaluée par électrophorèse) et un titre de 30 000 unités/mg (8 333 unités telles que mesurées selon la méthode de HOVINGH et al (1970), J. Biol. Chem. 245, 6, 1970).

2. Dépolymérisation de l'héparine

On dissout dans 50 ml d'eau distillée 1 g d'héparine pour utilisation thérapeutique. On ajoute 1 g d'acétate de sodium et 100 mg de chlorure de calcium. On ajuste le pH à 7,2 par addition de HCl 1 N.

On ajoute ensuite 1 mg d'une solution d'héparinase hautement purifiée telle qu'obtenue ci-dessus, et l'on soumet le mélange à incubation pendant 15 heures à 30 °C. Après précipitation par 10 volumes d'éthanol à 100° GL, on sèche le produit dépolymérisé obtenu. Ce produit sera désigné ci-après par P.

### B. Fractionnement sur « DEAE SEPHADEX A 25 »

Avec un tampon NaCl 0,1 M, pH 7,0 on équilibre une colonne de 16 mm de diamètre renfermant 50 ml de gel connu sous la dénomination indiquée sous B. On dépose 20 ml d'une solution renfermant 180 mg dudit produit P dans le tampon, puis on élue avec un gradient de NaCl selon lequel on utilise au départ 250 ml d'un tampon CaCl 0,1 M, puis d'un tampon $NaCl_2$ 0,5 M, pH 7,0. La vitesse d'élution est ajustée à 30 ml/h. On récupère des volumes successifs de 10 ml.

Sur la figure 1 on a représenté le diagramme d'élution suivi par mesure de la densité optique à 232 nm. Les volumes sont réunis en fractions 1, 2, 3, 4 selon les mesures de densité optique, comme montrées sur la figure 1. Leurs densités optiques respectives sont données dans le tableau 1 qui suit.

Tableau I

| No fraction | Poids | Rendement en poids % à partir de 120,8 mg de produit de départ | $[\alpha]_D^{20}$ (c=1, eau) sur la bande D du sodium | 232 nm DO.$_{H_2O}$ (conc: 1 mg/ml) |
|---|---|---|---|---|
| 1 | 22 mg | 18 % | + 33° | 2,1 |
| 2 | 69 mg | 57 % | + 30° | 3,2 |
| 3 | 20 mg | 16 % | + 30° | 2,6 |
| 4 | 9,8mg | 8 % | + 21° | 1,7 |

C. Chromatographie d'affinité sur agarose — AT III de la deuxième fraction

Avec un tampon NaCl tris-HCl 0,025 M, pH 7,4, on équilibre une colonne ayant un diamètre de 2,5 cm contenant 50 ml d'agarose, sur laquelle sont immobilisées des molécules d'AT III (Agarose-AT III).

On dissout dans 6 ml du tampon en question 60 mg de la fraction n° 2 (P 2) du tableau I, puis on dépose la solution au sommet de la colonne. On effectue l'élution avec des solutions successives :

Le premier éluant utilisé est le même que celui indiqué ci-dessus (on sépare ainsi un effluent contenant 50 mg du produit désigné par P 2 (B) contenu dans le premier pic détecté de densité optique à 232 nm) ; en changeant d'éluant et en utilisant NaCl 0,2 M tris-HCl 0,05 M, de pH 7,4, on ne désorbe aucune fraction active du gel ; en utilisant ensuite comme éluant $CaCl_2$ 2 M on obtient un pic (les volumes renfermant les fractions qui donnent naissance à ce pic sont précipités par de l'alcool et fournissent 5 mg de fraction à activité élevée (produit désigné par P 2 (A)).

Les caractéristiques physiques et biologiques de cette dernière fraction sont les suivantes :

titre YIN-WESSLER : 1 400 unités/mg

$[\alpha]_{D\,235\ nm}^{20} = + 24°$ (c = 1, eau)

DO.$_{HCl\ 0,03\ N}$ = 4,5

Les résultats de l'analyse de P 2 (A) sont donnés ci-dessous :

1. Analyse chimique

On dose les constituants de P 2 (A) en ce qui concerne :

— les acides uroniques selon la méthode de Bitter T. et al., Anal. Biochem., *4* (1962), 330-334.

— les hexosamines, d'une part par la méthode de Elson-Morgan, telle que modifiée par Antonopoulos C.A. et al., Biochem. Biophys. Acta *83* (1964), 1-19 (après hydrolyse de P 2 (A) par HCl 6N pendant 4 heures, à 110 °C sous atmosphère d'azote), et d'autre part par la méthode de Smith R. L. et Gilkerson E., Anal. Biochem., *98* (1979), 478-480, sur des échantillons soit non hydrolysés, soit hydrolysés de P 2 (A) ;

— les sulfates, par la méthode de T. T. Terho et al., Anal. Biochem., *41* (1971), 471-476.

Les résultats relatifs à la composition sont résumés dans le tableau II qui suit, exprimés en pourcentages en poids (première ligne), en rapport molaire par rapport aux motifs glucosamine après hydrolyse ($H^+$) selon la technique de Smith et Gilkerson (deuxième ligne).

Tableau II

| | Acides uroniques | Hexosamines | | Sulfates |
|---|---|---|---|---|
| | | Smith-Gil. $H^{(+)}$ | Antonopoulos | |
| P 2 (A) | 15,2 % | 18,7 % | 12,8 % | 12 % |
| | 0,9 | 1,0 | 0,7 | 1,4 |

Ces résultats montrent que les oligosaccharides de l'invention comprennent essentiellement :

— 1 mole d'acide uronique par mole d'hexosamine ;

— 1,4 mole de sulfate par motif disaccharidique (comportant un motif uronique et un motif hexosamine) ;

— des motifs 2 N-sulfate-glucosamine par motif 1 N-acétyl-glucosamine.

2. Chromatographie liquide sous haute pression

50 µl d'une solution de 0,5 mg/ml de P 2 (A) sont soumis à une élution à l'aide de $Na_2SO_4$ 0,02 M (1 ml/minute) à travers une première puis une deuxième colonne (250 × 4,6 cm) contenant respectivement de la silice commercialisée sous la dénomination LICHROPHOSPHER Si 100 et une troisième colonne (250 × 3 cm) remplie d'un matériau commercialisé (par WATERS et Associates) sous la dénomination micro-BONDAGEL. Le poids moléculaire de la fraction selon l'invention est apprécié en fonction du temps d'élution (ou de rétention sur les colonnes), en se référant aux variations linéaires des temps de rétention de polystyrène-sulfonates standards de poids moléculaires connus (respectivement 4 000, 6 500, 16 000 et 31 000) ainsi qu'avec un tétrasaccharide ayant un poids moléculaire connu de 700 à 900 soumis aux mêmes déterminations, exprimé en valeur logarithmique correspondant auxdits poids moléculaires. Les mesures ont été effectuées sur un chromatographe ® SPECTRAPHYSICS 3 500, et la détection des fractions par spectrophotométrie UV (200 nm et 230 nm).

Cette détermination ainsi que les autres déterminations chimiques selon la méthode de A. Linker et P. Hovingh, Biochem. volume 11, n° 4, 1972, p. 563-567, confirme que les oligosaccharides actifs ne renferment pas plus de 8 motifs saccharidiques.

3. Absorption en ultra-violet

On effectue les mesures d'absorption UV sur une solution de 100 µg/ml de P 2 (A), dans une bande UV de 215 à 260 nm. L'absorption maximale est observée à 230-235 nm.

Exemple 3

Procédé d'obtention de fractions oligosaccharidiques comprenant les étapes de :

I. Dépolymérisation enzymatique de l'héparine ;

II. Isolement des produits biologiquement actifs par chromatographie sur Sépharose-AT III ;

III. Gel filtration des fractions éluées et récupération des produits désirés.

I. Dépolymérisation enzymatique de l'héparine

On dissout 2 g d'héparine pour utilisation thérapeutique dans 100 ml d'un tampon acétate (NaOAc 0,15 M, NaCl 0,15 M ; $CaCl_2$ 0,005 M ; pH 6,9).

On soumet la solution à incubation pendant 24 heures à 30 °C.

L'héparinase hautement purifiée précédemment obtenue est ajoutée à la solution comme suit :

0,4 mg (quantité basée sur le dosage de protéines) au temps t = 0,

0,2 mg au temps t = + 8 heures,

0,2 mg au temps t = + 24 heures.

A t = + 36 heures, une autre addition d'héparinase ne provoque pas une augmentation de la densité optique à 232 nm. On considère que la réaction est terminée et que les produits sont récupérés par précipitation alcoolique et séchés (rendement en pourcentage en poids 90 %).

2. Chromatographie d'affinité sur Sépharose-AT III des produits dépolymérisés

On équilibre avec un tampon NaCl 0,1 M, tris-HCl 0,05 M, pH 7,5 une colonne de chromatographie (5 × 18 cm) contenant 10 mg d'AT III immobilisée par ml de Sépharose.

On dépose 2 g des produits de dégradation de l'héparine en haut de la colonne.

On lave la colonne avec ledit tampon, ce qui permet d'éliminer les fractions non fixées (les produits correspondants seront désignés ci-après par UP).

Les produits fixés (désignés par F P) sont ensuite élués avec une solution de $CaCl_2$ 1 M pH 7,2.

Les produits sont précipités avec de l'alcool et récupérés par centrifugation. On récupère d'une part 1,6 g de UP, et d'autre part 8 mg de FP ayant une activité (Yin-Wessler) de 800 u/mg.

3. Gel filtration

Les produits UP et FP sont soumis séparément à un gel filtration. 25 mg de chacun des produits sont filtrés sur une colonne (200 × 0,6 cm) d'un gel de Sephadex G 50 Superfine.

On réalise l'élution à l'aide d'une solution de NaCl 0,2 M. On recueille des fractions de 0,65 ml. Après élimination des sels sur Sephadex G 25, on lyophilise les produits.

Sur la figure 2 on a représenté les diagrammes d'élution des fractions UP (courbe en continu) et des fractions FP (courbe en pointillés).

L'élution est contrôlée par mesure de la densité optique à 230 nm (longueur d'onde d'absorption de la double liaison créée par l'héparinase).

Conformément aux mesures de densité optique, on fractionne UP en di-, tetra-, hexa-, et octasaccharides ($UP_2$, $UP_4$, $UP_6$ et $UP_8$) et en un autre produit ayant un degré de polymérisation supérieur ($UP_{+8}$). $UP_8$ et $UP_{+8}$ n'apparaissent pas sur les courbes d'élution de la figure 2 car ils représentent un trop faible pourcentage (3,4 et 1,3 % respectivement) et sont seulement détectés à sensibilité élevée.

La fraction FP est séparée en quatre fractions, désignées respectivement par FPa, b, c, et d, selon le volume décroissant d'élution. La majeure partie des fractions FP est éluée légèrement avant la fraction $UP_6$.

Dans le tableau 3, ci-après, on rapporte les résultats concernant chacune des fractions UP et FP.

Ces résultats comprennent : le volume d'élution (ml) de chacune desdites fractions provenant des fractions UP et FP ; le pourcentage de chacune de ces fractions dans le mélange des fractions respectivement UP et FP : le pouvoir rotatoire spécifique de chacune des fractions, (mesuré avec un polarimètre électronique en solution aqueuse; généralement à 1 %) ; l'absorbance à 230 mn (mesurée dans une solution à 0,01 % de HCl 0,03 M, les résultats étant exprimés en densité optique d'une solution 1 pour 1 000 ; et l'activité anti-Xa dans le plasma humain des fractions FP, mesurées selon le test de Yin-Wessler dont question ci-dessus.

(Voir tableau p. 14)

Tableau

| Fraction | Volume d'élution (ml) | % dans le mélange | $[\alpha]_D^{20}$ (c = 1, eau) sur la bande D du sodium | DO. 230 nm $H_2O$ | Activité anti-Xa u /mg Yin-Wessler |
|---|---|---|---|---|---|
| UP 2 | 52-60 | 52 | + 5 | 6 | - |
| UP 4 | 47-52 | 30,5 | + 23,5 | 4,8 | - |
| UP 6 | 43-47 | 13 | + 37 | 3,3 | - |
| UP 8 | 40-43 | 3,4 | + 41,5 | 2,5 | - |
| UP +8 | 37-40 | 1,3 | | 2 | |
| FPa | 40-45 | 50 | + 39 | 3,2 | 1200 (2000 dans d'autres tests) |
| FPb | 35-39 | 23 | + 41 | 2,5 | 930 |
| FPc | 27-34 | 14 | - | - | 200 |
| FPd | 23-26 | 5 | - | - | 330 |

Il ressort des résultats donnés dans le tableau ci-dessus que chacune des fractions FP possède une activité anti-Xa, l'activité la plus importante apparaissant dans la fraction FPa. On notera que cette fraction représente 50 à 60 % du mélange FP.

La fraction FPa a été soumise à plusieurs traitements afin d'élucider sa structure. Les résultats analytiques suivants ont été obtenus :

Dégradation de FPa par traitement avec de l'acide nitreux

Les fractions FPa récupérées après la gel filtration sont incubées avec $HNO_2$ en milieu aqueux dans les conditions permettant la dégradation des chaînes d'oligosaccharides. La dégradation est réalisée selon la méthode de Shiveley et Conrad, décrite dans Biochemistry 1976, 15, 3932-3942.

Sous l'action de $HNO_2$, les chaînes oligosaccharidiques sont fragmentées en di- et tétrasaccharides, la coupure intervenant après les motifs N-sulfate glucosamine, ces motifs étant transformés en groupes, 2,5-anhydromannose.

On sépare par chromatographie sur Séphadex G50 les di- et tétrasaccharides ainsi obtenus (200 × 0,5 cm ; NaCl 0,2M).

Le diagramme d'élution est donné sur la figure 3.

On effectue les mesures suivantes sur chacune de ces fractions ; la densité optique à 230 nm (courbe a) la quantité d'acide uronique (courbe b), de 2,5-anhydromannose (courbe c) et de glucosamine (avant et après hydrolyse acide), la radioactivité lorsque les produits analysés ont été tritiés avant d'être dégradés par $HNO_2$.

La mesure de la densité optique des fractions montre que la majeure partie des molécules insaturées sont des disaccharides, 90 % de la densité optique apparaissant dans le pic du disaccharide tandis que 10 % apparaissent dans le pic du fragment tétrasaccharidique.

On peut alors considérer que l'unité tétrasaccharidique ne renferme pas de motifs acide uronique avec une double liaison. Un tel motif fait partie du disaccharide qui contient en outre une N-sulfateglucosamine, ce disaccharide GH étant localisé à l'extrémité non réductrice d'oligosaccharide avant sa dégradation nitreuse. En outre, en dosant les groupes 2,5-anhydro-mannose dans ces fractions (courbe c dans la figure 3), on trouve 33 % des groupes 2,5-anhydro-mannose dans les fragments tétrasaccharidiques et 66 % dans les fragments disaccharidiques. En considérant que les chaînes oligosaccharidiques sont terminées par des groupes N-sulfate-glucosamine, on peut conclure de ces résultats que la fraction FPa contient deux chaînes disaccharidiques pour une chaîne tétrasaccharidique (voir courbe b sur la figure 3). Ces résultats sont confirmés par le dosage des acides uroniques (courbe b) selon Bitter et Muir dans Annal. Biochem. 1962, 4, 330-334 et des motifs glucosamine dans les produits dégradés provenant de FPa : ces produits de dégradation comprennent deux fois plus de motifs disaccharidiques que de motifs tétrasaccharidiques.

Réduction de la fraction FPa par du borohydrure de sodium suivie par une dégradation nitreuse

On réduit la fraction FPa avant dégradation nitreuse. Ainsi les extrémités réductrices des chaînes ne sont pas transformées en groupes 2,5-anhydro-mannose durant la dégradation nitreuse.

On effectue la réduction avec un tampon de borohydrure de sodium à pH 9,5. les extrémités réductrices des chaînes oligosaccharidiques FPa sont alors transformées en hexitols tritiés. Le produit réduit est séparé des sels présents dans le mélange par filtration sur Séphadex G25. On le soumet ensuite à une dégradation par de l'acide nitreux, puis à une gel filtration comme décrit ci-dessus. Le dosage des groupes 2,5-anhydro-mannose est alors réalisé et montre une diminution nette de ces groupes dans la fraction disaccharidique tandis que les fractions tétrasaccharidiques restent pratiquement inchangées.

En réduisant avec du borohydrure tritié, 70 à 80 % de la radioactivité est retrouvée dans les disaccharides et 20 à 30 % dans les tétrasaccharides. En outre, on observe qu'après ce traitement, la quantité de 2,5-anhydro-mannose diminue beaucoup plus dans le disaccharidique que dans le pic tétrasaccharidique : un tel résultat est en faveur de la présence d'un fragment disaccharidique à l'extrémité réductrice (70 % des molécules) et aussi du tétrasaccharide (30 %).

Dégradation par l'acide nitreux de la fraction FPa sous des conditions très ménagées

La fraction FPa est soumise à une dégradation par de l'acide nitreux dans des conditions très ménagées.

Il est alors possible, après gel filtration et chromatographie d'affinité, d'isoler une fraction oligosaccharidique contenant principalement les deux hexasaccharides possédant les séquences ABCDEF et CDEFGH, ces dernières faisant également partie de l'invention.

Dans ce procédé, on soumet FPa à l'action de l'acide nitreux selon la méthode décrite par CIFONELLI et KING (Carbohydrate Res. 1972, 21, 173-186), la réaction étant toutefois arrêtée après une à cinq minutes, de préférence au bout de trois minutes.

On élimine les sels des fragments ainsi obtenus par gel filtration sur Séphadex G25 puis on soumet les fractions recueillies à une chromatographie d'affinité dans les conditions décrites ci-dessus.

Les méthodes analytiques décrites ci-dessus pour l'étude de la fraction FPa, montrent qu'on dispose dans les fractions éluées de deux espèces principales, à savoir CDEFGH et ABCDEF. Ces fractions possèdent encore une activité anti-Xa (Yin-Wessler).

Caractérisation par RMN d'une fraction octasaccharidique

On isole une fraction octasaccharidique en opérant comme décrit ci-dessus. Cette fraction présente un titre Yin-Wessler de 2 000 unités/mg et un titre APTT de 4 ui/mg.

Le spectre RMN $C^{13}$ de ce produit est donné sur la figure 5. Il confirme que le produit est un octasaccharide. Il confirme également qu'il possède la structure attribuée avec la séquence ABCDEFGH.

Les signaux observés sont respectivement caractéristiques de :

— un carbone anomère en position 1 dans les différents motifs (ABCDEFG et H) de la structure (le motif correspondant étant mentionné pour chaque pic sur la figure pour permettre son identification 90-105 ppm environ),

— des carbones en position 6 (signal $C_6$) env. 60 et 70 ppm et en position 2 (signal $C_2$) des motifs glucosamines (55-60 ppm),

— $CH_3$ du groupe -NHAc dans D (environ 25 ppm).

En outre, on observe la présence d'un signal de résonance dans la région C-2 des résidus N-sulfate-amino-glucosamine (signal x), ce signal ne correspondant à aucun signal de résonance des spectres RMN obtenus en opérant dans des conditions similaires avec une héparine usuelle.

Exemple 4

Isolement d'une fraction hexasaccharidique active vis-à-vis du facteur Xa

Dans une autre série d'expériences, le mélange obtenu, après l'étape de chromatographie d'affinité, (80 mg) est chromatographié sur une colonne (200 × 2,5 cm) d'un gel de Séphadex G50 superfine.

On réalise l'élution avec du chlorure de sodium 0,2 M. Les produits sont détectés par absorption U.V. à 232 nm (voir figure 4).

La majeure partie du produit est éluée dans la région octasaccharidique et présente les mêmes propriétés que le produit précédemment décrit.

La fraction hexasaccharidique est réunie et les sels sont éliminés. Le produit ainsi obtenu est séché par congélation, le rendement est de 2 mg.

Ce composé présente une haute activité Yin-Wessler de 510 u/mg. Son titre APTT est de 3 ui/mg.

Etant donné que cette fraction hexasaccharidique est obtenue après dégradation par l'héparinase, elle contient les résidus A et H caractérisés dans la fraction octasaccharidique. En outre, étant donné que ce produit présente une affinité pour l'AT III, il doit contenir la séquence tétrasaccharidique CDEF. Il peut donc être représenté par la structure ABCDEF.

On ne peut cependant exclure la présence dans ce matériau de faibles quantités d'un produit ayant la structure CDEFGH où C est un résidu d'acide hexuronique insaturé (le groupe 2-OH étant sulfaté ou non).

L'étude analytique de cette fraction hexasaccharidique effectuée comme décrit ci-dessus pour l'octasaccharide (à savoir dégradation par l'acide nitreux et étude des fragments) — indique la présence à la fois de ABCDEF et CDEFGH.

Exemple 5

Dégradation de l'octasaccharide ABCDEFGH et oligosaccharides obtenus

1. En opérant conformément à la méthode décrite par L.A. Fransson dans Carbohydrate Research 62, 235-244, 1979 : on soumet l'octasaccharide ABCDEFGH à une réaction d'oxydation à l'aide de periodate de sodium en milieu phosphate, à pH 7 à 37 °C pendant 14 heures environ. On porte ensuite le pH à 11-12 par addition de soude pour provoquer une hydrolyse alcaline puis on neutralise le mélange réactionnel. Une chromatographie sur gel de Séphadex G-50 permet de séparer un trisaccharide auquel on peut attribuer la structure FGH.

L'étude de l'activité anti-Xa, selon le test Yin-Wessler permet de mettre en évidence une activité de l'ordre de 100 à 200 u/mg selon les essais.

2. On laisse agir une héparitinase purifiée, extraite de Flavobacterium heparinum sur l'octasaccharide dont question ci-dessus. Les conditions opératoires notamment de pH, température, durée, correspondent à celles mises en œuvre dans la dégradation enzymatique selon l'exemple 2. Par filtration sur un gel de Séphadex G-50, effectuée comme dans l'exemple 2, on récupère, à partir du mélange de dépolymérisation, deux tétrasaccharides auxquels on peut attribuer les structures EFGH et ABCD.

Le passage de ces produits sur une colonne de Sépharose AT III permet de retenir EFGH tandis que ABCD est éliminé. On récupère ensuite EFGH par élution avec une solution NaCl 1 M. L'activité Yin-Wessler mesurée dans différents essais est de l'ordre de 100 à 200 u/mg.

3. On soumet l'octasaccharide ABCDEFGH à une dégradation nitreuse ménagée. On met 1 mg

d'octasaccharide par ml de solution et on produit de l'acide nitreux (à une concentration voisine de 1 m M) in situ par addition de nitrite de sodium N/1 000 et de HCl. On ajuste le pH à 3. Au bout de 10 mn, à température ambiante, on ajuste le pH à 7. Le mélange réactionnel est soumis à une opération de gel filtration suivie d'une chromatographie d'affinité selon les conditions décrites dans les exemples précédents.

Par élution avec une solution de NaCl 1M ou de $CaCl_2$ 1M, on recueille un produit auquel on peut attribuer une structure hexasaccharidique CDEFGH.

Cet hexasaccharide est soumis à l'action d'une α-L-iduronidase, extraite de rein humain. Cette étape de dégradation enzymatique est réalisée à un pH 7 à 37 °C par filtration du mélange de dépolymérisation sur une colonne de Séphadex G5-0, on isole un oligosaccharide auquel on peut attribuer la structure d'un pentasaccharide DEFGH. L'activité Yin-Wessler de ce produit apparaît être de l'ordre de 400 u/mg dans les tests réalisés.

## Exemple 6

### Caractérisation par RMN de la fraction oligosaccharidique obtenue dans l'exemple 1

Le spectre RMN ($C^{13}$) de cette fraction montre la présence d'un signal X dans la région du C-2 des motifs glucosamine (voir figure 6). Ce signal peut être attribué à une glucosamine qui est N-sulfatée et substituée en position 3 par un groupe $—OSO_3^-$.

Ce motif glucosamine est sulfaté ou non en position 6.

En outre, la courbe d'intégration confirme que le produit possède un nombre moyen de motifs inférieur à 8 et comprend une majeure partie d'espèces hexasaccharidiques et octosaccharidiques.

En partant d'héparine de langue de bœuf, après dégradation contrôlée, suivie d'une chromatographie d'affinité et d'une gel filtration comme décrit ci-dessus, on peut isoler un octasaccharide ayant la structure ABCFGHGH, ce produit est hautement actif dans les tests anti-Xa.

On extrait également, en faible quantité, un hexasaccharide ABCFGH, qui s'avère également actif dans les essais anti-Xa.

L'invention s'étend naturellement aux oligosaccharides qui peuvent être obtenus par d'autres techniques de dépolymérisation de l'héparine ou de composés hépariniques, suivies par les étapes de récupération des oligosaccharides à faible poids moléculaire ayant une activité anticoagulante mesurable par le test Yin-Wessler.

Comme exemple d'autres techniques de dépolymérisation de l'héparine ou de composés proches, on peut citer l'oxydation périodique. On peut également citer la technique qui consiste à provoquer une réaction d'α,β-élimination par voie chimique sur l'héparine ou des esters d'héparine, donnant des résultats similaires, ou le procédé qui comprend :

— la mise en contact d'une fraction d'héparine avec de l'AT III immobilisée sur un gel de Sépharose afin de fixer les constituants de la fraction d'héparine capables de se fixer à l'AT III,

— la digestion des constituants de l'héparine ainsi fixés, par une héparinase, notamment d'origine bactérienne et,

— l'élution du gel des fragments à affinité par l'AT III, le traitement de l'éluant renfermant lesdits fragments avantageusement selon les étapes décrites ci-dessus.

## Revendications

1. Fractions oligosaccharidiques possédant une activité anti-Xa (Yin-Wessler), pouvant être obtenues par un procédé qui comprend les étapes de :

— mise en contact de l'héparine (ou des fractions hépariniques) possédant une activité anticoagulante et comportant des chaînes d'un poids moléculaire d'environ 2 000 à 50 000, avec un agent capable de dépolymériser ou fragmenter les chaînes hépariniques, les conditions utilisées pour réaliser cette étape étant ajustées de manière à obtenir un mélange de dépolymérisation qui contient des fragments (ou chaînes oligosaccharidiques courtes) constitués par huit motifs au plus, possédant cependant une activité anti-Xa (Yin Wessler) d'au moins 100 u/mg alors que leur activité anticoagulante globale (titre USP) est faible par rapport à celle de l'héparine, voire pratiquement nulle, et possédant une affinité élevée pour l'AT III,

— le contact du mélange de dépolymérisation avec de l'AT III pour absorber ou retenir au moins la majeure partie, avantageusement pratiquement la totalité des oligosaccharides possédant une séquence ayant une affinité spécifique vis-à-vis de l'AT III,

— la récupération des produits fixés sur l'AT III et,

— l'isolement des fractions recherchées renfermant lesdites chaînes oligosaccharidiques constituées par huit motifs au plus, et les sels pharmaceutiquement acceptables de ces fractions.

2. Fractions oligosaccharidiques selon la revendication 1, caractérisées par le fait que les produits récupérés à partir du procédé mentionné ci-dessus sont soumis à une ou plusieurs étapes afin de séparer les oligosaccharides actifs à chaînes courtes définis ci-dessus, et avantageusement sont fractionnés, par

exemple, par gel perméation selon leur poids moléculaire et/ou leur densité ionique, les fractions désirées étant ensuite récupérées.

3. Fractions oligosaccharidiques selon la revendication 1, caractérisées par le fait que, dans ledit procédé, le mélange de dépolymérisation est fractionné avant d'être mis en contact avec l'AT III.

4. Fractions oligosaccharidiques selon l'une quelconque des revendications 1 à 3, caractérisées par le fait que, dans ledit procédé, le matériau héparinique est dépolymérisé par voie chimique, en particulier, avec de l'acide nitreux, $HNO_2$, en milieux aqueux, ce qui entraîne la formation de chaînes saccharidiques contenant à leur extrémité réductrice un groupe 2,5-anhydromannose.

5. Fractions oligosaccharidiques selon l'une quelconque des revendications 1 à 3, caractérisées par le fait que, dans ledit procédé, le matériau héparinique est dépolymérisé par voie enzymatique, avantageusement avec une héparinase hautement purifiée, plus spécialement une héparinase d'origine bactérienne, ce qui entraîne la formation de chaînes saccharidiques terminées à leur extrémité non réductrice par un acide uronique $\alpha,\beta$-insaturé.

6. Fractions d'oligosaccharides selon l'une quelconque des revendications 1 à 5, caractérisées en ce qu'elles possèdent un titre Yin-Wessler d'au moins 700 u/mg.

7. Fractions d'oligosaccharides selon la revendication 6, caractérisées en ce qu'elles possèdent un titre Yin-Wessler d'au moins 1 200 u/mg et un titre USP très faible, voire pratiquement nul.

8. Fractions d'oligosaccharides selon la revendication 7, caractérisées en ce qu'elles possèdent un titre Yin-Wessler compris entre 1 200 et 2 000 u/mg.

9. Chacun des oligosaccharides constituant les fractions selon l'une quelconque des revendications 1 à 8, *per se*.

10. Fractions d'oligosaccharides et leurs sels caractérisées en ce que ces oligosaccharides

1) comportent au plus 8 motifs

2) comprennent au moins un motif N-sulfate-D-glucosamine,

3) possèdent une activité anti-Xa d'au moins 10 fois celle de l'héparine (exprimée par le titre Yin-Wessler) et une activité anticoagulante globale (exprimée par le titre USP) très faible, voire pratiquement nulle,

4) une affinité spécifique élevée pour l'AT III,

5) un rapport du titre Yin-Wessler au titre USP d'au moins 30.

11. Oligosaccharides selon l'une quelconque des revendications précédentes, caractérisés en outre en ce qu'ils renferment au moins un motif saccharidique choisi dans le groupe comprenant les motifs de structure acide 2-O-sulfate 4,5-insaturé glucuronique, N-sulfate D-glucosamine éventuellement 3 et/ou 6-O-sulfate, acide L-iduronique, le cas échéant, 2-O-sulfate, N-acétyl D-glucosamine, le cas échéant, 6-O-sulfate et acide D-glucuronique.

12. Oligosaccharides selon la revendication 11, caractérisés en ce qu'ils comportent un motif de structure N-sulfate-D-glucosamine comportant un groupe sulfate en position 3.

13. Oligosaccharides selon la revendication 11 ou 12, caractérisés en ce qu'ils renferment du côté de l'extrémité réductrice un motif de structure N-sulfate-D-glucosamine, substitué en position 3 et/ou 6 par un groupe sulfate.

14. Oligosaccharides selon l'une quelconque des revendications 11 à 13, caractérisés en ce qu'ils renferment un motif de structure acide 2-O-sulfate uronique 4,5-insaturé du côté de l'extrémité non réductrice.

15. Oligosaccharides selon l'une quelconque des revendications 11 à 14, caractérisés par la présence d'environ une molécule de N-acétyl-D-glucosamine par chaîne oligosaccharidique.

16. Oligosaccharides selon l'une quelconque des revendications 11 à 15, caractérisés par la présence d'un motif N-acétyl-D-glucosamine pour deux ou trois motifs N-sulfate-D-glucosamine.

17. Oligosaccharides selon l'une quelconque des revendications 11 à 15, caractérisés en ce qu'ils contiennent une séquence de motifs saccharidiques choisie dans le groupe comprenant ABCDEFGH, ABGHCDEF, ABCDEF, CDEFGH et DEFGH, ces différents symboles présentant les significations suivantes :

A = acide uronique ou acide uronique insaturé,

B = N-sulfate-D-glucosamine ou N-sulfate 6-O-sulfate D glucosamine,

C = acide L-iduronique, le cas échéant, lorsque ce motif se trouve du côté de l'extrémité non réductrice, un acide iduronique insaturé,

D = N-acétyl D-glucosamine ou N-acétyl 6-O-sulfate D-glucosamine,

E = acide D-glucuronique,

F = N-sulfate 3-O-sulfate D-glucosamine ou N-sulfate 3-O-sulfate 6-O-sulfate D-glucosamine,

G = acide 2-O sulfate L-iduronique et

H = N-sulfate D-glucosamine ou N-sulfate 6-O sulfate D-glucosamine.

18. L'octasaccharide de structure ABCDEFGH selon la revendication 17, dans lequel A est un acide glucuronique et qui est terminé par un groupe 2, 5 anhydro-manno.

19. L'octasaccharide de structure ABCDEFGH selon la revendication 17, dans lequel A est un acide glucuronique insaturé.

20. Les sels de sodium, de calcium ou de magnésium des fractions d'oligosaccharides et des oligosaccharides selon l'une quelconque des revendications précédentes.

21. Fractions d'oligosaccharides et oligosaccharides selon l'une quelconque des revendications 10 à 20, caractérisées par des spectres RMN, relatifs au $^{13}C$, comprenant un signal dans la région du carbone anomère en position 2 des résidus N-sulfate-glucosamine.

22. Procédé d'obtention de fractions oligosaccharidiques selon l'une quelconque des revendications 1 à 10 ou 21, ou des oligosaccharides selon l'une quelconque des revendications 11 à 21, caractérisé en ce qu'il comprend :

a) la mise en contact de l'héparine (ou de fractions hépariniques) possédant une activité anticoagulante et comportant des chaînes avec des poids moléculaires d'environ 2 000 à environ 50 000, avec un agent capable de dépolymériser ou de fragmenter les chaînes hépariniques dans des conditions ajustées afin d'obtenir un mélange de dépolymérisation qui contient des fragments ou des chaînes oligosaccharidiques constituées par huit motifs au plus, possédant cependant une activité anti-Xa (Yin-Wessler) d'au moins 100 u/mg alors que leur activité anticoagulante globale est faible par rapport à celle de l'héparine, voire nulle, et une affinité spécifique élevée pour l'AT III ;

b) la mise en contact du mélange de dépolymérisation avec de l'AT III pour adsorber ou retenir au moins la majeure partie des oligosaccharides possédant une séquence ayant une affinité spécifique vis-à-vis de l'AT III,

c) la désorption des produits retenus sur l'AT III et la récupération des fractions et oligosaccharides recherchés.

23. Procédé selon la revendication 22, caractérisé en ce que l'héparine est dépolymérisée par voie chimique ou enzymatique.

24. Procédé selon la revendication 22, caractérisé en ce que la dépolymérisation de l'héparine est réalisée à l'aide de $HNO_2$ en milieu aqueux à un pH de l'ordre de 2 à 4, et à une température de l'ordre de l'ambiante.

25. Procédé selon la revendication 23, caractérisé en ce que la dépolymérisation de l'héparine est réalisée à l'aide d'une héparinase hautement purifiée d'origine bactérienne, à un pH de 6 à 8, et à une température de l'ordre de l'ambiante.

26. Procédé selon la revendication 25, caractérisé en ce que l'héparinase provient de Flavobacterium heparinum.

27. Procédé selon l'une quelconque des revendications 23 à 26, caractérisé en ce qu'on réalise la séparation de la majeure partie des produits contenus dans le mélange de dépolymérisation ayant une activité anti-Xa élevée, par chromatographie d'affinité sur une colonne contenant de l'AT III immobilisée, cette colonne étant équilibrée avec un tampon ayant une force ionique d'environ au moins 0,1 M, à un pH de 6 à 8, les produits dépourvus d'affinité pour l'AT III ou n'ayant qu'une faible activité pour l'AT III étant éliminés par lavage avec un tampon.

28. Procédé selon la revendication 27, caractérisé en ce que le tampon utilisé pour ladite élimination est du même type que celui utilisé pour équilibrer la colonne.

29. Procédé selon l'une quelconque des revendications 23 à 28, caractérisé en ce qu'il comprend, pour récupérer les produits retenus ou adsorbés sur l'AT III, ayant une activité anti-Xa (Yin-Wessler), des étapes de désorption de tous les oligosaccharides fixés par élution avec un tampon ayant une force ionique suffisante à cet effet, le tampon utilisé pour cette élution étant choisi parmi ceux qui n'interfèrent pas avec les étapes suivantes de récupération des oligosaccharides contenus dans les fractions récupérées.

30. Procédé selon l'une quelconque des revendications 23 à 29, caractérisé en ce qu'il comprend le fractionnement par gel perméation du mélange de tous les oligosaccharides précédemment retenus sur AT III, ce fractionnement étant avantageusement conduit afin d'obtenir tout d'abord l'élution des plus grosses molécules, puis celle des plus petites molécules, en partant des fractions qui possèdent un titre Yin-Wessler et un titre USP qui sont dans un rapport d'au moins 30, la récupération s'étendant à toutes les fractions restantes qui possèdent encore une activité anti-Xa au moins dans le test Yin-Wessler.

31. Procédé selon l'une quelconque des revendications 22 à 30, caractérisé en ce qu'avant l'étape de fixation sur AT III, on élimine du mélange de dépolymérisation les oligosaccharides possédant plus de huit motifs saccharidiques, avantageusement par un fractionnement tel que par gel filtration.

32. Procédé selon la revendication 23, 25 ou 26, caractérisé en ce que la dépolymérisation est réalisée en présence d'un tampon renfermant du calcium.

33. Les produits intermédiaires du groupe constitué par le mélange de dépolymérisation obtenu à l'issue de l'étape a) du procédé selon la revendication 22, ou des étapes de dépolymérisation visées dans l'une quelconque des revendications 23 à 26, les produits récupérés après fixation sur AT III selon l'étape b) du procédé selon la revendication 22, ou les étapes selon l'une quelconque des revendications 27 à 29, ou les produits récupérés, selon l'étape du procédé de la revendication 31, après fractionnement du mélange de dépolymérisation.

34. Composition pharmaceutique contenant une quantité efficace d'au moins un oligosaccharide selon l'une quelconque des revendications 12 à 22, ou d'une fraction selon l'une quelconque des revendications 1 à 11 ou 22 en association avec un véhicule pharmaceutique.

35. Composition selon la revendication 34, caractérisée en ce qu'elle est utilisée pour la prévention ou le traitement de thrombose et qu'elle se présente sous la forme d'une solution concentrée stérile injectable contenant de 1 000 à 100 000 u/ml (Yin-Wessler) environ d'oligosaccharides, de préférence de

5 000 à 50 000, notamment de 25 000 u/ml environ, lorsqu'elle est destinée à l'injection par voie sous-cutanée, ou contenant encore de 500 à 10 000 environ, notamment de 5 000 ui/ml d'oligosaccharides environ, lorsqu'elle est destinée à l'administration par voie intraveineuse ou par perfusion.

36. Composition pharmaceutique pour la prévention ou le traitement de thrombose, caractérisée en ce qu'elle comprend une quantité efficace d'au moins un oligosaccharide selon l'une quelconque des revendications 12 à 22, ou d'une fraction selon l'une quelconque des revendications 1 à 11 ou 22 en association avec un véhicule pharmaceutique.

**Claims**

1. Oligosaccharidic fractions having an anti-Xa (Yin-Wessler) activity, said oligosaccharidic fractions being of the type obtainable by a process which comprises the steps of :

— contacting heparin (or hepanic fractions) possessing anticoagulant activity and having chains with molecular weights ranging from about 2 000 to 50 000, with an agent capable of depolymerizing or fragmenting the heparinic chains, the conditions used for carrying out this step being adjusted so as to obtain a depolymerization mixture which contains oligosaccharidic fragments (or short oligosaccharidic chains), constituted by no more than 8 moieties yet having an anti-Xa activity (Yin-Wessler) of at least 100 u/mg while the whole anticoagulant activity (USP titer) is low with respect to that of heparin, even practically nil, and having a high affinity for AT III,

— contacting the depolymerization mixture with AT III to absorb or retain at least the major part, advantageously nearly all the totality of the oligosaccharides having a sequence with specific affinity with respect to AT III,

— recovering the products fixed on AT III and,

— isolating the searched fractions containing said oligosaccharidic chains constituted by no more than 8 moieties, and the pharmaceutically acceptable salts of the said fractions.

2. Oligosaccharidic fractions according to claim 1, characterized by the fact that the products recovered from the above mentioned process are submitted to one or several steps before separating the above defined active oligosaccharides with short chains, and advantageously are fractionated, for example by gel permeation according to their molecular weight and/or their ionic density, the desired fractions being then recovered.

3. Oligosaccharidic fractions according to claim 1, characterized by the fact that, in the said process, the depolymerization mixture is fractionated before being brought into contact with AT III.

4. Oligosaccharidic fractions according to any one of claims 1, 2 and 3, characterized by the fact that, in the said process, the heparinic material is depolymerized in a chemical way, particularly, with nitrous acid, $HNO_2$, in an aqueous medium, which results in the formation of saccharidic chains having at their reducing end a 2,5-anhydromannose group.

5. Oligosaccharidic fractions according to any one of claims 1, 2 and 3, characterized by the fact that, in the said process, the heparinic material is depolymerized in an enzymatic way, advantageously by highly purified heparinase, and more especially bacterial heparinase, resulting in the formation of saccharidic chains terminated at their non-reducing end by an α,β-unsaturated uronic acid.

6. Oligosaccharidic fractions according to any one of claims 1 to 5, characterized in that they have a Yin-Wessler titer of at least 700 u/mg.

7. Oligosaccharidic fractions according to claim 6, characterized in that they have a Yin-Wessler titer of at least 1,200 u/mg and a very low USP titer, even practically nil.

8. Oligosaccharidic fractions according to claim 7 characterized in that they have a Yin-Wessler titer between 1 200-2 000 u/mg.

9. Each of the oligosaccharides constituting the fractions according to any one of claims 1 to 8, *per se.*

10. Oligosaccharidic fractions and the salts thereof, characterized in that the said oligosaccharides :

1) comprise at most 8 moieties,

2) comprise at least one N-sulfate-D-glucosamine moiety,

3) have an anti-Xa activity of at least ten times that of heparine (expressed by the Yin-Wessler titer) and a whole anticoagulant activity (expressed by the USP titer), very low, even practically nil,

4) a high specific activity for AT III,

5) a ratio of the Yin-Wessler titer to the USP titer of at least 30.

11. Oligosaccharidic fractions according to any one of the preceding claims, further characterised by the fact that they contain at least one saccharidic moiety selected from the group comprising moieties of 2-O sulfate 4,5-unsaturated glucuronic acid, N-sulfate-D-glucosamine optionally 3 and/or 6-O-sulfate, L-iduronic acid, optionally 2-O sulfate, N-acetyl D-glucosamine, optionally 6-O sulfate and D-glucuronic acid structure.

12. Oligosaccharides according to claim 11, characterized in that they contain moiety having an N-sulfate-D-glucosamine structure comprising a sulfate group at position three.

13. Oligosaccharides according to claims 11 or 12, characterized in that they contain at the reducing end a moiety having the N-sulfate-D-glucosamine structure, substituted at 3 and/or 6 positions by a sulfate group.

14. Oligosaccharides according to any one of claims 11 to 13, characterized in that they contain moiety having 2-O-sulfate uronic acid structure 4,5-unsaturated at the non-reducing end.

15. Oligosaccharides according to any one of claims 11 to 14, characterized by the presence of about one molecule of N-acetyl-D-glucosamine per oligosaccharidic chain.

16. Oligosaccharides according to any one of claims 11 to 15, characterized by the presence of one N-acetyl-D-glucosamine moiety for two or three N-sulfate-D-glucosamine moieties.

17. Oligosaccharides according to any one of claims 11 to 15, characterized in that they contain a sequence of saccharidic moieties selected from the group comprising ABCDEFGH, ABGHCDEF, ABCDEF, CDEFGH and DEFGH, the different symbols having the following meanings :

A = uronic acid or unsaturated uronic acid,
B = N-sulfate-D-glucosamine or N-sulfate-6-O-sulfate-D-glucosamine,
C = L-iduronic acid or, when present at a chain end, unsaturated uronic acid,
D = N-acetyl-D-glucosamine or N-acetyl-6-O-sulfate-D-glucosamine
E = D-glucuronic acid,
F = N-sulfate-3-O-sulfate-D-glucosamine or N-sulfate-3-O-sulfate-6-O-sulfate-D-glucosamine,
G = 2-O-sulfate-L-iduronic acid, and
H = N-sulfate-D-glucosamine or N-sulfate-6-O-sulfate-D-glucosamine.

18. The octasaccharide having an ABCDEFGH structure according to claim 17, wherein A is glucuronic acid and which is terminated by a 2,5-anhydromanno group.

19. The octasaccharide having an ABCDEFGH structure according to claim 17 wherein A is an unsaturated glucuronic acid.

20. The sodium, calcium or magnesium salts of the oligosaccharidic fractions and of the oligosaccharides according to any one of the preceding claims.

21. Oligosaccharidic fractions and oligosaccharides according to any one of the claims 10 to 20, characterized by the NMR spectra, relating to $C^{13}$ comprising a signal in the anomeric carbon area at the two positions of the N-sulfate-glucosamine residues.

22. A process for obtaining oligosaccharidic fractions according to any one of claims 1 to 10 or 21, or the oligosaccharides according to any one of claims 11 to 21, characterized in that it comprises :

a) contacting heparin (or hepanic fractions) possessing an anticoagulant activity and having chains with molecular weights ranging from about 2 000 to 50 000, with an agent capable of depolymerizing or fragmenting the heparinic chains, the conditions used for carrying out that step being adjusted so as to obtain a depolymerization mixture which contains oligosaccharidic fragments or chains, constituted by non more than 8 moieties yet having an anti-Xa activity (Yin-Wessler) of at least 100 u/mg while the whole anticoagulant activity (USP titer) is low with respect to that of heparin, even practically nil, and having a high affinity for AT III,

b) contacting the depolymerization mixture with AT III for absorbing or retaining at least the major part, advantageously nearly all the totality of the oligosaccharides having a sequence with specific affinity with respect to AT III,

c) desorbing the products retained on AT III and recovering the search fractions and oligosaccharides.

23. Process according to claim 22, characterized by the fact that heparine is depolymerized in a chemical or enzymatic way.

24. Process according to claim 22, characterized in that heparin depolymerization is carried out with $HNO_2$ in an aqueous medium at a pH in the range of 2 to 4 and in an ambient temperature range.

25. Process according to claim 23, characterized in that heparine depolymerization is carried out with highly purified bacterial heparinase, at a pH 6-8 and in an ambient temperature range.

26. Process according to claim 25, characterized in that the heparinase originates from Flavobacterium heparinum.

27. Process according to any one of claims 23 to 26, characterized in that the major part of the products contained in the depolymerization mixture having an high anti-Xa activity is separated by chromatography affinity on a column containing immobilised AT III, the said column being equilibrated with a buffer having a ionic strength of about at least 0,1 M, at a pH 6-8, the products devoid of affinity for AT III or having activity only for AT III being eliminated by rinsing with a buffer.

28. Process according to claim 27, characterized in that the buffer used for the elimination is of the same type as that used for equilibrating the column.

29. Process according to any one of claims 23 to 28, characterized in that it comprises, for recovering the products retained or absorbed on AT III, having an anti-Xa activity (Yin-Wessler), the steps of desorbing all the oligosaccharides fixed by elution with a buffer having an ionic strength sufficient in that respect, the buffer used for the said elution being selected from those which do not interfere with the following steps of recovery of the oligosaccharides contained in the said recovered fractions.

30. Process according to any one of claims 21 to 29, characterized in that it comprises fractionating by gel permeation the mixture of all the oligosaccharides previously retained on AT III, the said fractionation being advantageously carried out so as to obtain first elution of the biggest molecules, and then that of the smallest molecules, starting from the fractions having Yin-Wessler titer and USP titer in a

ratio of at least 30, the recovery extending to all the remaining fractions still having an anti-Xa activity at least in the Yin-Wessler test.

31. Process according to any one of claims 22 to 30, characterized in that before the fixation step of AT III, the oligosaccharides having more than 8 saccharidic moieties are eliminated from the depolymerization mixture, advantageously by fractionation such as gel filtration.

32. Process according to claims 23, 25 or 26, characterized in that the depolymerization is carried out in the presence of a buffer containing calcium.

33. The intermediary products of the group constituted by the depolymerization mixture obtained at the end of step a) of the process according to claim 22, or the depolymerization steps disclosed in any one of claims 23 to 26, the products recovered after fixation on AT III according to step b) of the process according to claim 22, other steps according to any one of claims 27 to 29, other products recovered according to the process step of claim 31, after fractionation of the depolymerization mixture.

34. Pharmaceutical composition containing an effective amount of at least one oligosaccharide according to any one of claims 12 to 22 or of a fraction according to any one of claims 1 to 11 or 22 in association with a pharmaceutical carrier.

35. Composition according to claim 34, characterized in that it is used for preventing or treating thrombosis and that it is in the form of an injectable, sterile, concentrated solution, containing about 1 000 to 100 000 u/ml (Yin-Wessler) of oligosaccharides, preferably from 5 000 to 50 000, notably of about 25 000 u/ml, when it is destined for injection by a subcutaneous route, or containing again from about 500 to 10 000, notably about 5 000 u/ml of oligosaccharides, when it is destined for intra-veinous injection or for perfusion.

36. Pharmaceutical composition for preventing of treating thrombosis, characterized in that it comprises an effective amount of at least one oligosaccharide according to any one claims 12 to 22, or of a fraction according to any one of claims 1 to 11 or 22 in association with a pharmaceutical carrier.

**Patentansprüche**

1. Oligosaccharidfraktionen mit einer Anti-Xa(Yin-Wessler)-Aktivität, die nach einem Verfahren erhalten werden können, welches die folgenden Stufen umfaßt:

— Inberührungbringen von Heparin (oder von Heparinfraktionen) mit einer Antikoagulationsaktivität und mit Ketten, die ein Molekulargewicht von etwa 2 000 bis 50 000 aufweisen, mit einem zur Depolymarisation oder Spaltung der Heparinketten befähigten Mittel, wobei die zur Durchführung dieser Stufe angewendeten Bedingungen derart abgestimmt werden, daß ein Depolymerisationsgemisch erhalten wird, welches Oligosaccharidfragmente oder kurze Oligosaccharidketten enthält, die aus höchstens acht Abschnitten bestehen, dennoch aber eine Anti-Xa (Yin-Wessler)-Aktivität von wenigstens 100 Einheiten/mg besitzen, während ihre Gesamtantikoagulationsaktivität (USP-Titer) in bezug auf jene von Heparin gering oder sogar praktisch Null ist, und die eine hohe Affinität für AT III (Antithrombin III) aufweisen,

— das Inberührungbringen des Depolymerisationsgemisches mit AT III zur Absorption oder Zurückhaltung wenigstens des größten Teils, vorteilhafterweise praktisch der Gesamtheit der Oligosaccharide mit einer Sequenz, die eine spezifische Affinität für AT III aufweist,

— die Gewinnung der auf dem AT III fixierten Produkte, und

— die Isolierung der gewünschten Fraktionen, welche die genannten, aus höchstens acht Abschnitten bestehenden Oligosaccharidketten umfassen, und die pharmaseutisch annehmbaren Salze dieser Fraktionen.

2. Oligosaccharidfraktionen nach Anspruch 1, dadurch gekennzeichnet, daß die beim oben erwähnten Verfahren erhaltenen Produkte einer oder mehreren Stufen unterworfen werden, um die aktiven Oligosaccharide mit wie oben definierten kurzen Ketten abzutrennen, und vorteilhafterweise fraktioniert werden, beispielsweise aufgrund von Geldurchlässigkeit nach ihrem Molekulargewicht und/oder ihrer Ionendichte, wobei die gewünschten Fraktionen anschließend gewonnen werden.

3. Oligosaccharidfraktionen nach Anspruch 1, dadurch gekennzeichnet, daß das Depolymerisationsgemisch bei dem genannten Verfahren fraktioniert wird, bevor es mit dem AT III in Berührung gebracht wird.

4. Oligosaccharidfraktionen nach einem der Ansprüche 1 bis 3, dadurch gekennzeichnet, daß das Heparinmaterial beim genannten Verfahren auf chemischem Wege depolymerisiert wird, insbesondere mit Salpetriger Säure, $HNO_2$, in wässerigem Milieu, was zur Bildung von Saccharidketten führt, die an ihrem reduzierenden Ende eine 2,5-Anhydromannosegruppe enthalten.

5. Oligosaccharidfraktionen nach einem der Ansprüche 1 bis 3, dadurch gekennzeichnet, daß das Heparinmaterial bei dem genannten Verfahren auf enzymatischem Wege depolymerisiert wird, vorteilhafterweise mit einer hoch-gereinigten Heparinase, insbesondere einer Heparinase bakteriellen Ursprungs, was zur Bildung von Saccharidketten führt, die an ihrem nicht reduzierenden Ende durch eine α,β-ungesättigte Uronsäure abgeschlossen sind.

6. Oligosaccharidfraktionen nach einem der Ansprüche 1 bis 5, dadurch gekennzeichnet, daß sie einen Yin-Wessler-Titer von wenigstens 700 Einheiten/mg aufweisen.

7. Oligosaccharidfraktionen nach Anspruch 6, dadurch gekennzeichnet, daß sie einen Yin-Wessler-Titer von wenigstens 1 200 Einheiten/mg und einen sehr niedrigen USP-Titer, sogar einen solchen, der praktisch Null ist, aufweisen.

8. Oligosaccharidfraktionen nach Anspruch 7, dadurch gekennzeichnet, daß sie einen Yin-Wessler-Titer zwischen 1 200 und 2 000 Einheiten/mg aufweisen.

9. Jedes der Oligosaccharide, welche die Fraktionen nach einem der Ansprüche 1 bis 8 darstellen, als solches.

10. Oligosaccharidfraktionen und ihre Salze, dadurch gekennzeichnet, daß diese Oligosaccharide

1) höchstens 8 Abschnitte aufweisen,

2) wenigstens einen N-Sulfat-D-glucosaminabschnitt umfassen,

3) eine Anti-Xa-Aktivität von wenigstens dem Zehnfachen jener von Heparin (ausgedrückt durch den Yin-Wessler-Titer) und eine Gesamtantikoagulationsaktivität (ausgedrückt durch den USP-Titer), die sehr gering und sogar praktisch Null ist, besitzen,

4) eine hohe spezifische Affinität für AT III haben, und

5) ein Verhältnis des Yin-Wessler Titers zum USP-Titer von wenigstens 30 zeigen.

11. Oligosaccharide nach einem der vorhergehenden Ansprüche, weiterhin dadurch gekennzeichnet, daß sie wenigstens einen Saccharidabschnitt enthalten, der aus der Gruppe ausgewählt ist, welche die Abschnitte mit der Struktur von 4,5-ungesättigtem Glucuronsäure-2-O-sulfat, D-Glucosamin-N-sulfat, gegebenenfalls 3- und/oder 6-O-Sulfat, L-Iduronsäure, gegebenenfalls 2-O-Sulfat, N-Acetyl-D-glucosamin, gegebenenfalls 6-O-Sulfat, und D-Glucuronsäure umfaßt.

12. Oligosaccharide nach Anspruch 11, dadurch gekennzeichnet, daß sie einen Abschnitt mit der Struktur von D-Glucosamin-N-sulfat, der eine Sulfatgruppe in 3-Stellung trägt, umfassen.

13. Oligosaccharide nach Anspruch 11 oder 12, dadurch gekennzeichnet, daß sie auf der Seite ihres reduzierenden Endes einen Abschnitt mit der Struktur von D-Glucosamin-N-sulfat, das in Stellung 3 und/oder 6 durch eine Sulfatgruppe substituiert ist, enthalten.

14. Oligosaccharide nach einem der Ansprüche 11 bis 13, dadurch gekennzeichnet daß sie auf der Seite ihres nicht reduzierenden Endes einen Abschnitt mit der Struktur von 4,5-ungesättigtem Uronsäure-2-O-sulfat enthalten.

15. Oligosaccharide nach einem der Ansprüche 11 bis 14, gekennzeichnet durch das Vorliegen von annähernd einem Molekül N-Acetyl-D-glucosamin je Oligosaccharidkette.

16. Oligosaccharide nach einem der Ansprüche 11 bis 15, gekennzeichnet durch das Vorliegen eines N-Acetyl-D-glucosaminabschnittes je zwei oder drei Abschnitte von D-Glucosamin-N-sulfat.

17. Oligosaccharide nach einem der Ansprüche 11 bis 15, dadurch gekennzeichnet, daß sie eine Sequenz mit Saccharid Abschnitten enthalten, die aus der ABCDEFGH, ABGHCDEF, ABCDEF, CDEFGH und DEFGH umfassenden Gruppe ausgewählt sind, bei diese verschiedenen Symbole die folgenden Bedeutungen haben :

A = Uronsäure oder ungesättigte Uronsäure,

B = D-Glucosamin-N-sulfat oder D-Glucosamin-N-sulfat-6-O-sulfat,

C = L-Iduronsäure, gegebenenfalls, wenn sich der Abschnitt auf der Seite des nicht reduzierenden Endes befindet, eine ungesättigte Iduronsäure,

D = N-Acetyl-D-glucosamin oder N-Acetyl-D-glucosamin-6-O-sulfat,

E = D-Glucuronsäure,

F = D-Glucosamin-N-sulfat-3-O-sulfat oder D-Glucosamin-N-sulfat-3-O-sulfat-6-O-sulfat,

G = L-Iduronsäure-2-O-sulfat und

H = D-Glucosamin-N-sulfat oder D-Glucosamin-N-sulfat-6-O-sulfat.

18. Das Octasaccharid mit der Struktur ABCDEFGH nach Anspruch 17, in welcher A eine Glucuronsäure ist und welches durch eine 2,5-Anhydro-manno-Gruppe abgeschlossen ist.

19. Das Octasaccharid mit der Struktur ABCDEFGH nach Anspruch 17, in welcher A eine ungesättigte Glucuronsäure ist.

20. Die Natrium-, Calcium- oder Magnesiumsalze von Oligosaccharidfraktionen und von Oligosacchariden nach einem der vorhergehenden Ansprüche.

21. Oligosaccharidfraktionen und Oligosaccharide nach einem der Ansprüche 10 bis 20, gekennzeichnet durch KMR-Spektren, bezogen auf $^{13}C$, die ein Signal im Bereich der Anomerkohlenstoffes in Stellung 2 der Glucosamin-N-sulfatreste aufweisen.

22. Verfahren zur Gewinnung von Oligosaccharidfraktionen nach einem der Ansprüche 1 bis 10 oder 21, oder von Oligosacchariden nach einem der Ansprüche 11 bis 21, dadurch gekennzeichnet, daß es umfaßt :

a) das Inberührungbringen von Heparin (oder von Heparinfraktionen) mit einer Antikoagulationsaktivität und mit Ketten, die Molekulargewichte von etwa 2 000 bis etwa 50 000 aufweisen, mit einem zur Depolymerisation oder Spaltung der Heparinketten befähigten Mittel, und zwar unter derart eingestellten Bedingungen, daß ein Depolymerisationsgemisch erhalten wird, welches Oligosaccharidfragmente oder -ketten enthält, die aus höchstens acht Abschnitten bestehen, dennoch aber eine Anti-Xa (Yin-Wessler)-Aktivität von wenigstens 100 Einheiten/mg besitzen, während ihre Gesamtantikoagulationsaktivität in bezug auf jene von Heparin gering oder sogar Null ist, und die eine hohe spezifische Affinität für AT III aufweisen ;

b) das Inberührungbringen des Depolymerisationsgemisches mit AT III zur Absorption oder Zurückhaltung wenigstens des größten Teiles der Oligosaccharide mit einer Sequenz, die eine spezifische Affinität für AT III aufweist,

c) die Desorption der auf dem AT III zurückgehaltenen Produkte und die Gewinnung der gewünschten Oligosaccharidfraktionen.

23. Verfahren nach Anspruch 22, dadurch gekennzeichnet, daß das Heparin auf chemischem oder enzymatischem Wege depolymerisiert wird.

24. Verfahren nach Anspruch 22, dadurch gekennzeichnet, daß die Depolymerisation von Heparin mit Hilfe von $HNO_2$ in wässerigem Milieu bei einem pH im Bereich von 2 bis 4 und bei einer Temperatur im Bereich von Zimmertemperatur bewirkt wird.

25. Verfahren nach Anspruch 23, dadurch gekennzeichnet, daß die Depolymerisation von Heparin mit Hilfe einer hoch-gereinigten Heparinase bakteriellen Ursprungs bei einem pH von 6 bis 8 und bei einer Temperatur im Bereich von Zimmertemperatur bewirkt wird.

26. Verfahren nach Anspruch 25, dadurch gekennzeichnet, daß die Heparinase von Flavobacterium heparinum stammt.

27. Verfahren nach einem der Ansprüche 23 bis 26, dadurch gekennzeichnet, daß man die Abtrennung des Hauptteils der Produkte, welche in dem eine hohe Anti-Xa-Aktivität aufweisenden Depolymerisationsgemisch enthalten sind, durch Affinitätschromatographie an einer das immobilisierte AT III enthaltenden Säule vornimmt, wobei diese Säule mit einem Puffer ins Gleichgewicht gebracht worden ist, der eine lonenstärke von wenigstens etwa 0,1 M bei einem pH von 6 bis 8 aufweist, wobei die Produkte, die für das AT III keine Affinität aufweisen oder für das AT III eine nur geringe Aktivität zeigen, durch Waschen mit einem Puffer entfernt werden.

28. Verfahren nach Anspruch 27, dadurch gekennzeichnet, daß der für die genannte Entfernung verwendete Puffer vom gleichen Typus ist wie jener, der für das Insgleichgewichtbringen der Säule verwendet worden ist.

29. Verfahren nach einem der Ansprüche 23 bis 28, dadurch gekennzeichnet, daß es zur Gewinnung der auf dem AT III zurückgehaltenen oder adsorbierten Produkte, die eine Anti-Xa-(Yin Wessler)-Aktivität aufweisen, die Stufen der Desorption aller fixierten Oligosaccharide durch Eluierung mit einem Puffer, der eine für diesen Zweck ausreichende lonenstärke hat, umfaßt, wobei der für diese Eluierung verwendete Puffer unter jenen ausgewählt ist, welche die folgenden Stufen der Gewinnung der in den gewonnenen Fraktionen enthaltenen Oligosaccharide nicht stören.

30. Verfahren nach einem der Ansprüche 23 bis 29, dadurch gekennzeichnet, daß es die Fraktionierung der Mischung aller Oligosaccharide, die vorher auf AT III zurückgehalten worden sind, mittels Geldurchlässigkeit umfaßt, wobei diese Fraktionierung vorteilhafterweise durchgeführt wird, um zuerst die größten Moleküle und dann die kleinsten Moleküle eluiert zu erhalten, wobei von Fraktionen ausgegangen wird, die einen Yin-Wessler-Titer und einen USP-Titer aufweisen, welche Titer in einem Verhältnis von wenigstens 30 stehen, und die Gewinnung sich auf alle verbleibenden Fraktionen erstreckt, die wenigstens im Yin-Wessler-Test noch eine Anti-Xa-Aktivität besitzen.

31. Verfahren nach einem der Ansprüche 22 bis 30, dadurch gekennzeichnet, daß man vor der Stufe der Fixierung auf AT III vom Depolymerisationsgemisch, vorteilhafterweise durch eine Fraktionierung, wie durch Gelfiltration, die Oligosaccharide entfernt, die mehr als 8 Saccharidabschnite besitzen.

32. Verfahren nach Anspruch 23, 25 oder 26, dadurch gekennzeichnet, daß die Depolymerisation in Gegenwart eines Calcium enthaltenden Puffers ausgeführt wird.

33. Die Zwischenprodukte der Gruppe, bestehend aus dem Depolymerisationsgemisch, das am Ende der Stufe a) des Verfahrens nach Anspruch 22 erhalten wird, oder der Depolymerisationsstufen, die in einem der Ansprüche 23 bis 26 vorgesehen sind, die Produkte, welche nach Fixierung auf AT III gemäß Stufe b) des Verfahrens nach Anspruch 22, oder gemäß den Stufen nach einem der Ansprüche 27 bis 29 gewonnen werden, oder die Produkte, die gemäß der Stufe des Verfahrens von Anspruch 31 nach Fraktionierung des Depolymerisationsgemisches gewonnen werden.

34. Pharmazeutische Zusammensetzung, enthaltend eine wirksame Menge wenigstens eines Oligosaccharids nach einem der Ansprüche 12 bis 22, oder einer Fraktion nach einem der Ansprüche 1 bis 11 oder 22, gemeinsam mit einem pharmazeutischen Träger.

35. Zusammensetzung nach Anspruch 34, dadurch gekennzeichnet, daß sie zur Vorbeugung oder Behandlung von Thrombose verwendet wird und daß sie in Form einer sterilen, konzentrierten Injektionslösung vorliegt, welche etwa 1 000 bis 100 000 Einheiten/ml (Yin-Wessler), vorzugsweise 5 000 bis 50 000, insbesondere etwa 25 000 Einheiten/ml Oligosaccharide, wenn sie für eine subkutane Injektion bestimmt ist, oder aber etwa 500 bis 10 000, insbesondere etwa 5 000 i.E./ml Oligosaccharide, falls sie zur Verabreichung auf intravenösem Wege oder durch Perfusion bestimmt ist, enthält.

36. Pharmazeutische Zusammensetzung zur Vorbeugung oder Behandlung von Thrombose, dadurch gekennzeichnet, daß sie eine wirksame Menge wenigstens eines Oligosaccharids nach einem der Ansprüche 12 bis 22, oder einer Fraktion nach einem der Ansprüche 1 bis 11 oder 22, gemeinsam mit einem pharmazeutischen Träger, enthält.

Fig.1.

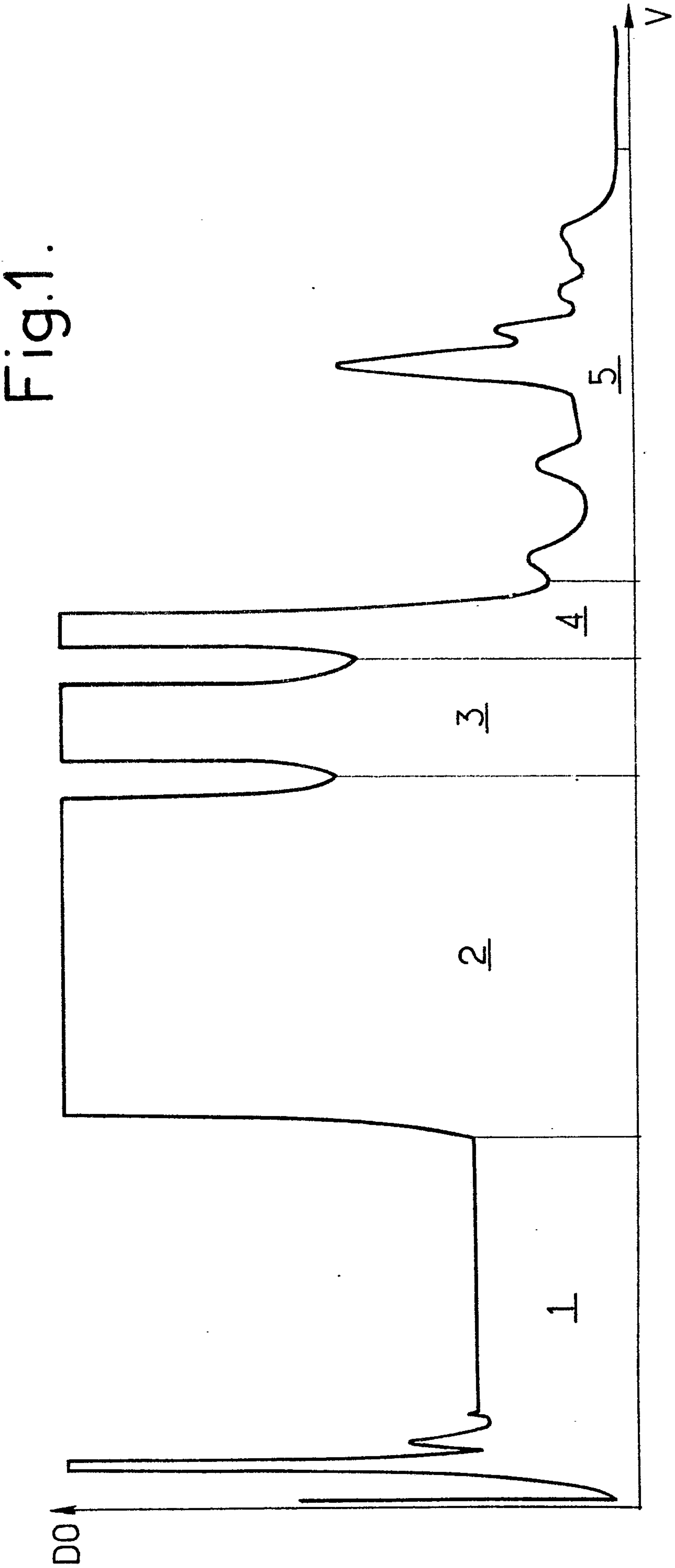

Fig.2.

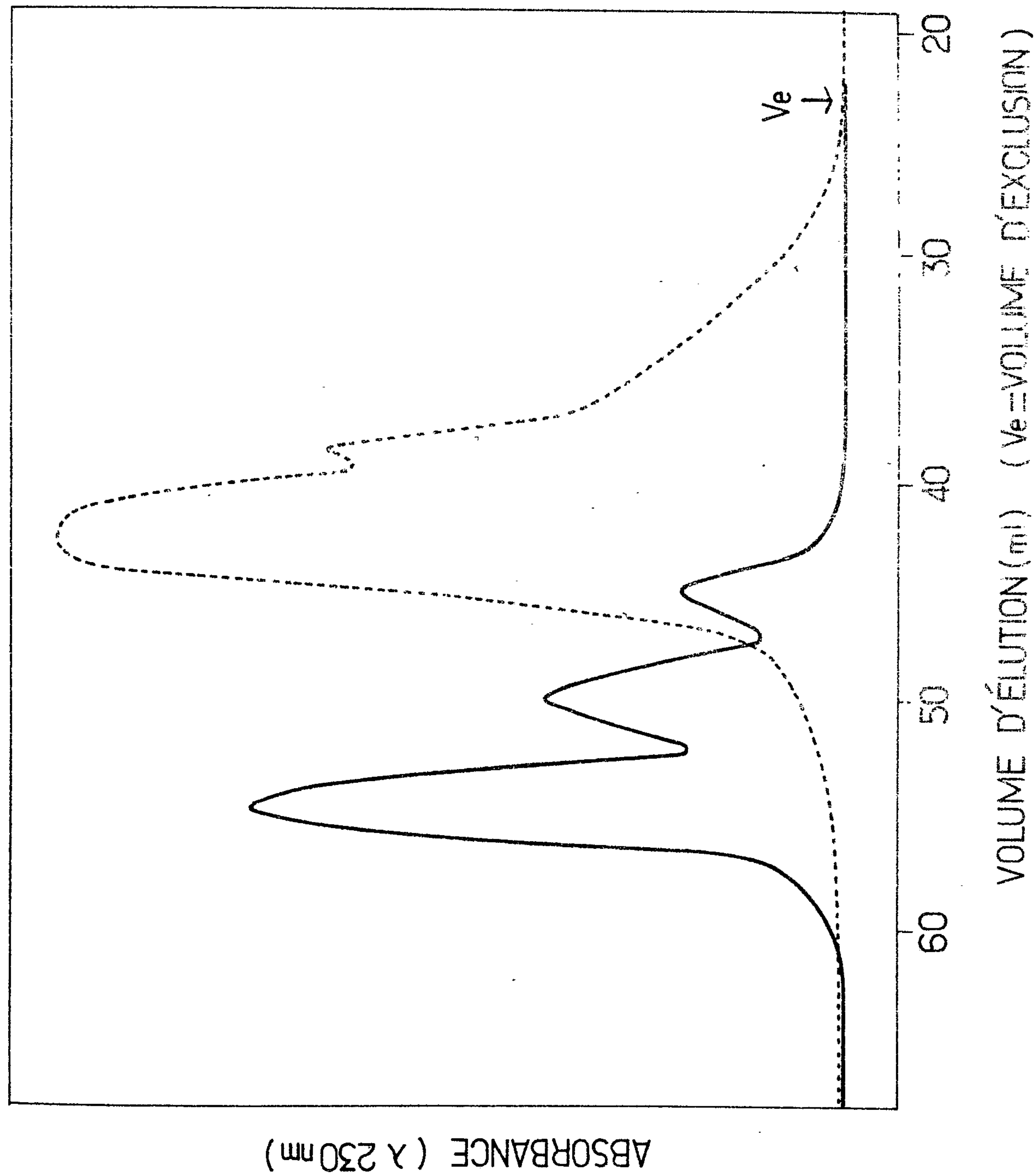
Ve
20
30
40
50
60
VOLUME D'ÉLUTION (ml) (Ve = VOLUME D'EXCLUSION)
ABSORBANCE (λ 230 nm)

Fig.3.

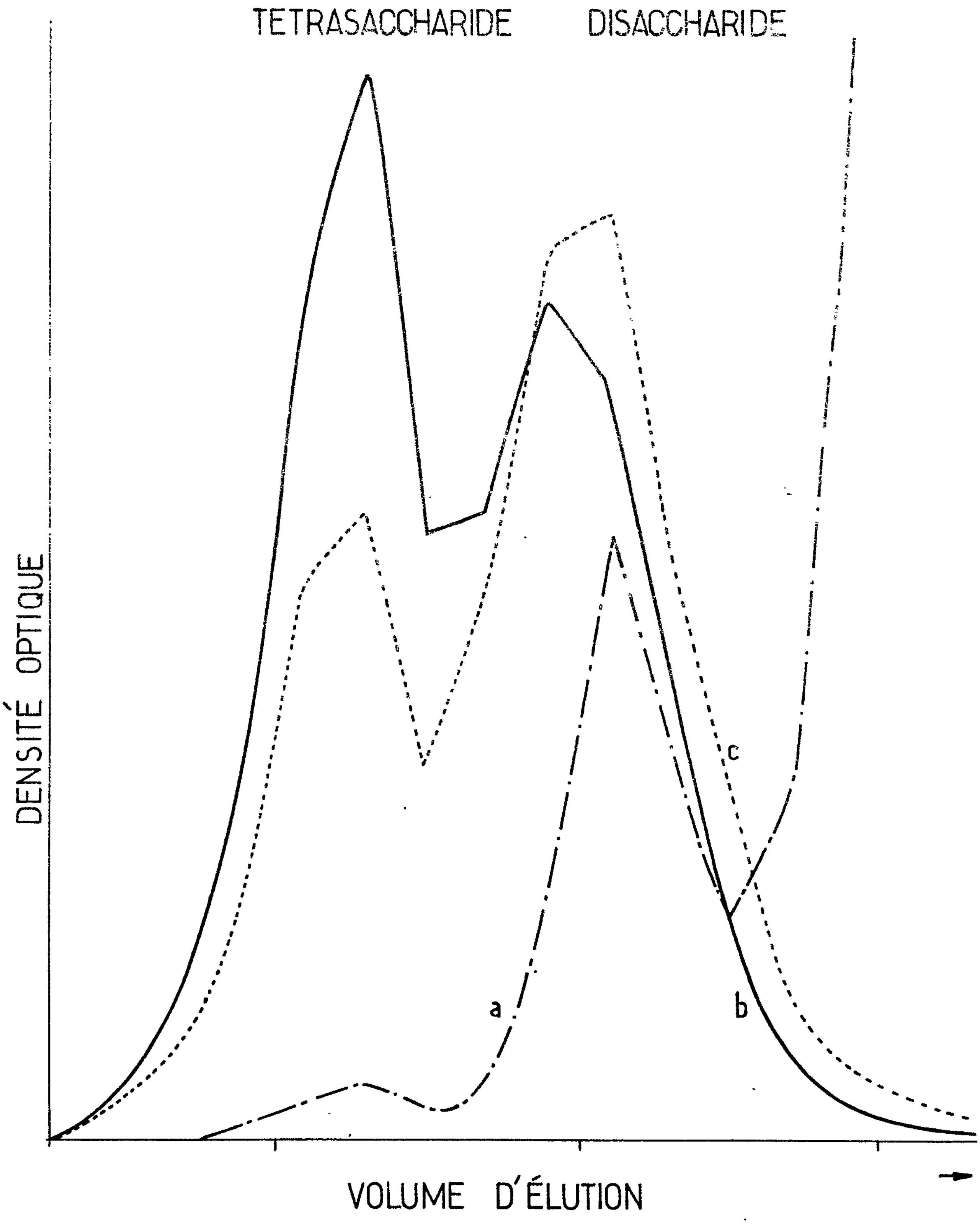
TETRASACCHARIDE
DISACCHARIDE
DENSITÉ OPTIQUE
c
a
b
VOLUME D'ÉLUTION

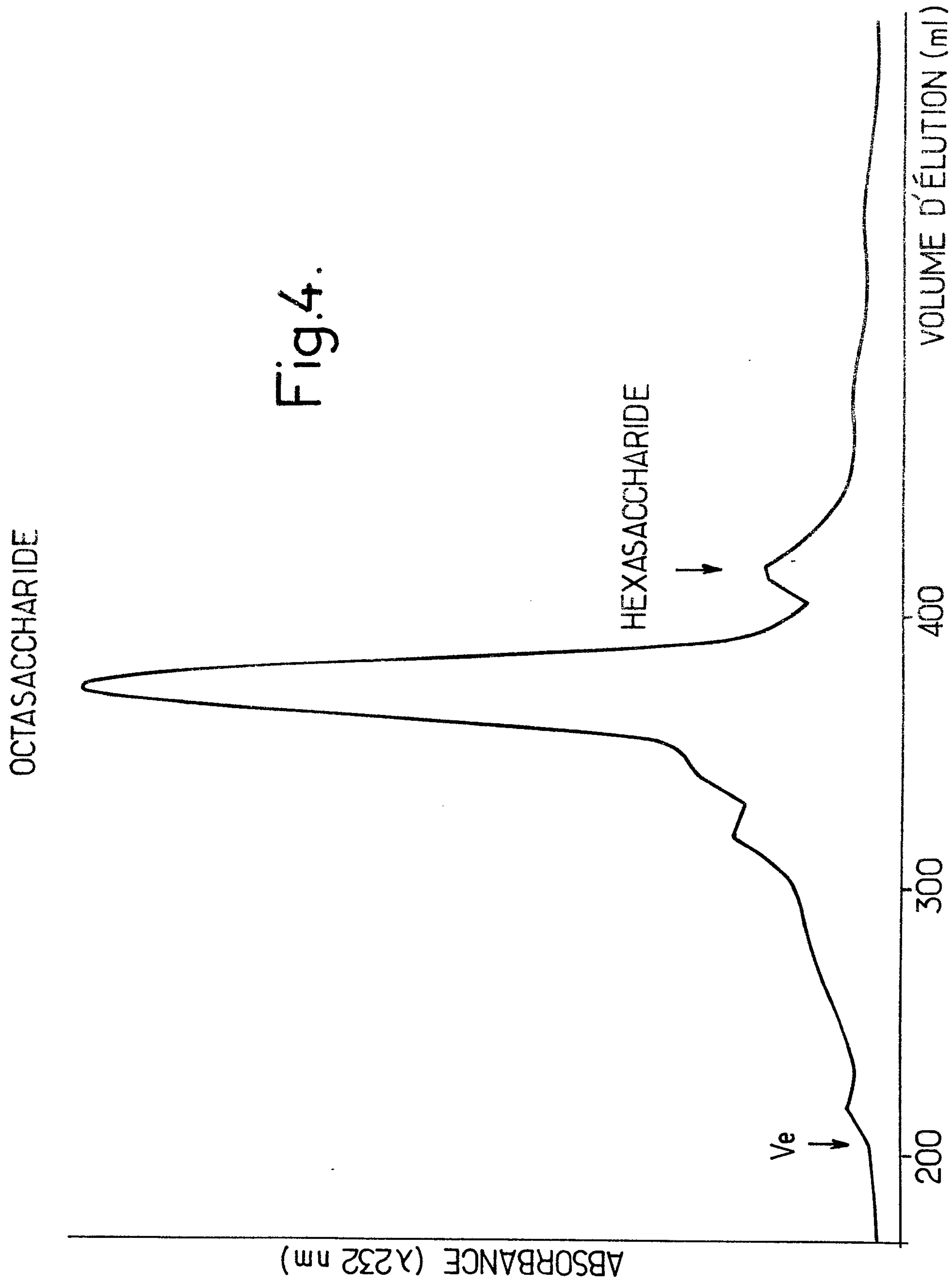
OCTASACCHARIDE
HEXASACCHARIDE
Ve
ABSORBANCE (λ232 nm)
VOLUME D'ÉLUTION (ml)
200
300
400

Fig.4.

Fig.5

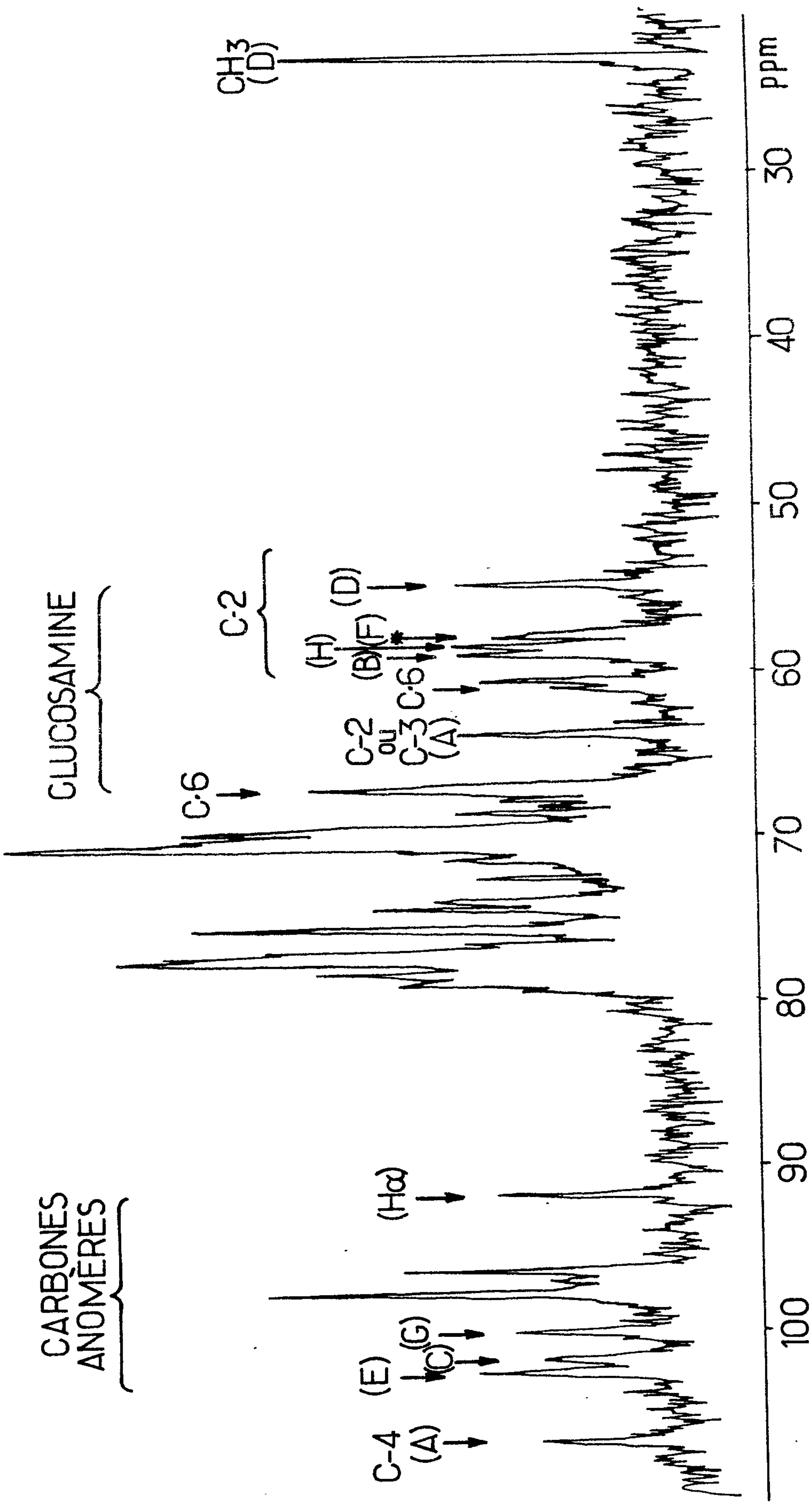
CARBONES ANOMÈRES
GLUCOSAMINE
C-4 (A)
(E)
(C)
(G)
(Hα)
C-6
C-2 ou C-3 (A)
C-6
(B)(F)
(H)
(D)
C-2
CH3 (D)
ppm
100
90
80
70
60
50
40
30

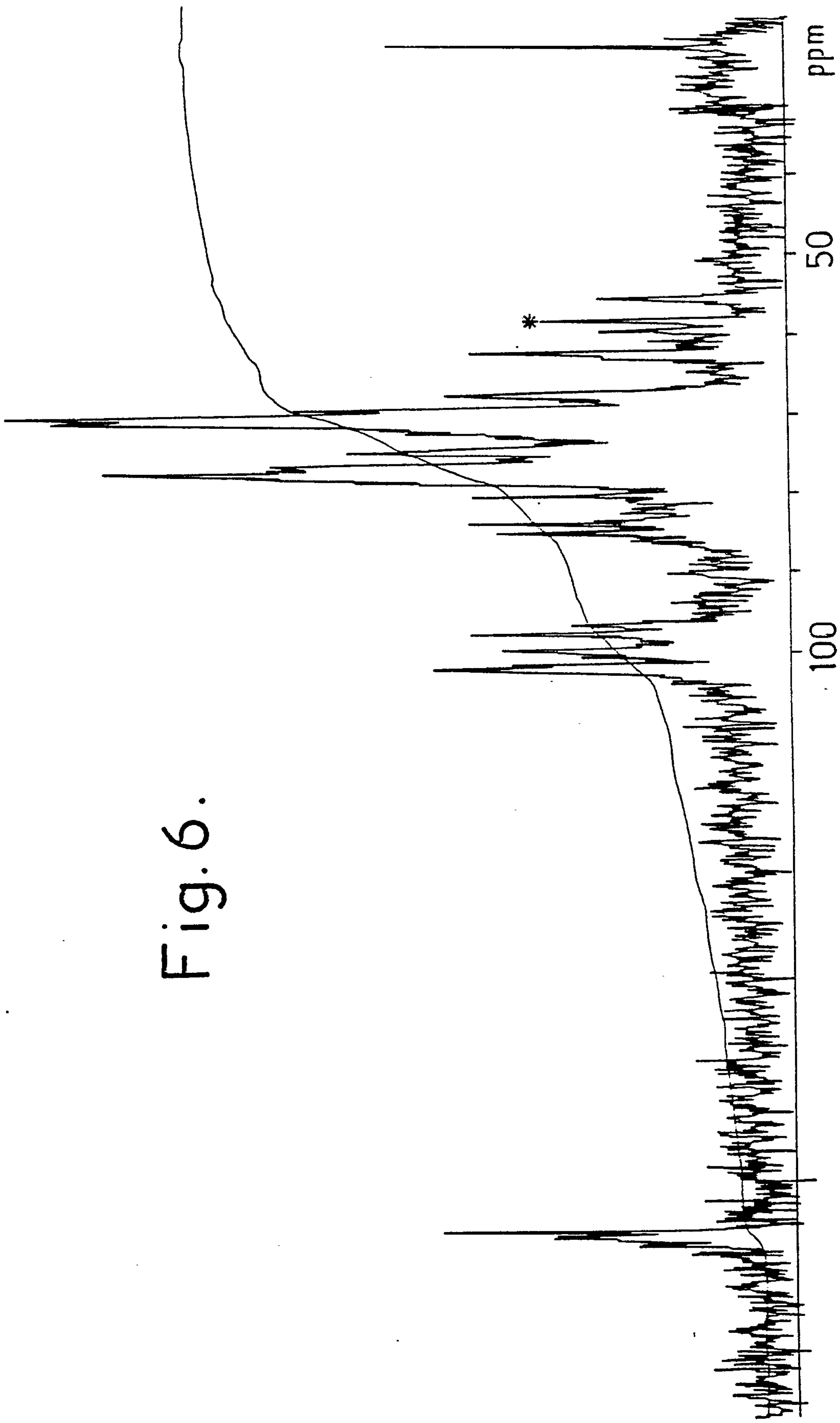


Fig. 6.